(19) **Europäisches Patentamt**
**European Patent Office**
**Office européen des brevets**

(11) **EP 2 648 490 A1**

(12) **EUROPEAN PATENT APPLICATION**
published in accordance with Art. 153(4) EPC

(43) Date of publication:
**09.10.2013 Bulletin 2013/41**

(51) Int Cl.:
*H05H 6/00* (2006.01)     *A61N 5/10* (2006.01)
*G21K 5/08* (2006.01)     *H05H 3/06* (2006.01)
*H05H 9/00* (2006.01)

(21) Application number: **11844142.7**

(22) Date of filing: **29.11.2011**

(86) International application number:
**PCT/JP2011/077557**

(87) International publication number:
**WO 2012/073966 (07.06.2012 Gazette 2012/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.11.2010   JP 2010264724
04.03.2011   JP 2011048187
20.05.2011   JP 2011113950
02.09.2011   JP 2011192030**

(71) Applicant: **Inter-University Research Institute
Corporation
High Energy Accelerator Research Organization
Tsukuba-shi
Ibaraki 305-0801 (JP)**

(72) Inventors:
• **MATSUMOTO, Hiroshi**
**Tsukuba-shi**
**Ibaraki 305-0801 (JP)**
• **KOBAYASHI, Hitoshi**
**Tsukuba-shi**
**Ibaraki 305-0801 (JP)**
• **YOSHIOKA, Masakazu**
**Tsukuba-shi**
**Ibaraki 305-0801 (JP)**

(74) Representative: **Vigand, Philippe et al
Novagraaf International SA
3 chemin de l'Echo
1213 Onex Geneva (CH)**

(54) **COMBINED-TYPE TARGET, NEUTRON GENERATING METHOD USING COMBINED-TYPE
TARGET, AND NEUTRON GENERATING APPARATUS USING COMBINED-TYPE TARGET**

(57)     A target is provided herein such that the radio-activation of a member thereof due to protons may be reduced. In order to reduce the radioactivation of the member due to protons, a novel target composed by compositing a beryllium material (or lithium material) and a nonmetal material is used.

FIG. 1

**Description**

FIELD

The present invention relates to a target to generate neutrons by colliding protons with the target, a neutron generating method use of a target and a neutron generating apparatus by used of a target. More specifically, it is an object to provide a novel target for addressing heat problems related to the target and reducing radioactivation of a target member and the like due to protons and neutrons, a neutron generating method by use of this target, and a neutron generating apparatus by use of this target.

BACKGROUND

Recently, researches and developments are increasing for methods and apparatuses for producing neutrons for Boron Neutron Capture Therapy (BNCT), which is expected to be a selective cancer therapy. These methods and apparatuses are disclosed in patent documents 1 and 2, for example.

Patent document 1 features that a deuteron beam from, for example, 30 MeV to 40 MeV generated by a radio frequency quadrupole linac (RFQ linac) is collided with lithium to cause an Li (d, n) reaction to produce neutrons, and then using a neutron moderator to produce thermal neutrons and epithermal neutrons for the therapy.

Patent document 2 relates to a target for producing neutrons and features that tungsten covered with Nb, Pt, Au, Al, Be, Cr, stainless steel or an alloy thereof, which is a low-hydrogen absorber, is used for improving corrosion resistance against a coolant for a target which is collided with a high-intensity proton beam.

Patent document 3 features that neutrons are produced by colliding heavy hydrogen ion beam with a surface of liquid lithium or an alloy of the lithium and a metal which acts as a catalyst for fusion reaction to induce a cold fusion reaction.

Patent document 4 features that a proton beam with energy more than or equal to 20 MeV generated by a cyclotron and the like is collided with heavy metal such as tantalum and tungsten and the like to produce neutrons including a nuclear spallation material, and eliminate from the neutrons the harmful nuclear spallation material and fast neutrons via a filter constructed of a neutron moderator unit and lead to produce thermal neutrons and epithermal neutrons for the therapy.

Patent document 5 discloses a method and an apparatus for producing neutrons on the basis of a fixed field alternating gradient (FFAG) - emittance recovery internal target (ERIT) system. In addition, patent document 5 features that a proton beam or a deuteron beam with energy from more than or equal to 11 Mev and less than 15 MeV which is subject to circling enhancement by a cyclotron-type proton storage ring is collided with a beryllium target set in the ring to produce neutrons, and then modulated through a moderator such as heavy water to obtain thermal neutrons and epithermal neutrons for the therapy.

Patent document 6 discloses a metal target which is collided with a proton beam with energy more than or equal to about 11 Mev and output more than or equal to about 30 kW which is accelerated by an RFQ linac or a drift tube linac to produce neutrons. In addition, patent document 6 discloses that the metal target is preferably beryllium. Further, patent document 6 features that the thickness of the target is almost the same with or slightly larger than the range of the proton beam in the target, and that the target is cooled via a metal plate which has a heat transfer area larger than or equal to the heat transfer area of the target.

Patent Document 7 features that a linear accelerator is used to collide a proton beam with, for example, 11 MeV with a beryllium target to generate fast neutrons with more than or equal to 10 keV, and then the fast neutrons are filtered through a moderator such as heavy water to be modulated into epithermal neutrons with less than 10 keV or thermal neutrons with less than or equal to 0.5 eV.

Patent document 8 features that a method of crimping a rolled lithium film onto a copper substrate is a method of manufacturing a lithium target.

Patent document 9 features a lithium target for generating neutrons by colliding a proton with energy slightly larger than the threshold for Li(p, n) reaction (about 2 MeV) with the target. In addition, patent document 9 features that the target structure for preventing lithium from melting is a structure in which a cone-shaped incision is made in a block having a cooling mechanism and a lithium film covered with beryllium on a backing foil is applied to the surface of the cone-shaped incision.

Patent document 10 features a lithium target for generating neutrons. In addition, patent document 10 features that the structure of lithium particles for preventing the lithium particles from melting and preventing liquefied lithium from leaking is a structure in which the lithium particles are covered sequentially with sintered carbon, silicon carbide and zirconium carbide.

Patent document 11 features a lithium target for BNCT. In addition, patent document 11 features that the lithium target is lithium which is attached onto an iron substrate, a tantalum substrate or a vanadium substrate.

Patent document 12 features a lithium target for generating neutrons by colliding a proton beam with output of from 20 mA to 50 kW and energy of about 2.5 MeV with the target. In addition, patent document 12 features that the target structure for preventing lithium from melting is a structure in which a palladium film is applied onto the surface of a cone-shaped heat transfer plate having a cooling mechanism and a lithium film is attached onto the palladium film.

However, the methods and apparatuses in patent documents 1 to 7 as described above require that a proton beam or a deuteron beam to be collided with a target should have acceleration energy of 11 MeV, which means a high energy proton beam. Therefore, the methods and the apparatuses disclosed in the patent documents 1- 7 given above have the following problems in terms of practical use. That is, a large- sized accelerator for generating the proton beams or the heavy proton beams is required. Conspicuous radioactation of the member such as the target is caused by the high- energy proton beams. A large- sized cooling device is required for cooling the target. It is hard to handle the target in the case of a liquid target. In the case of a solid- state target, a comparatively thick target material for preventing the target from being melted is adhered onto a metallic support member having thermal conductivity. When the target material for generating the neutrons is made of a metal such as a heavy metal, the metal is mixed with a considerable amount of fast neutrons that are extremely harmful to a human body and have high radioactation of the members of the apparatus, and hence there is required a large scale decelerating apparatus for decelerating the primarily generated neutrons. A special safe management system is needed for absorbing or removing the harmful and highly radioactive proton beams, neutrons and nuclear reaction secondary substances. An embrittlement preventive measure of the target material due to active hydrogen as a reaction by- product should be taken. Especially, the problem of the radioactation by the member such as the target due to the proton beams and the neutrons is a problem of radiation exposure received from the radioactive member and is therefore the critical problem that should be solved.

Further, as seen in the patent document 6, in the case of using the solid- state target of the beryllium, it is indispensable to remove the heat generated at the target, and therefore such an idea is proposed as to enlarge a heat conduction area of the metallic support member for supporting the target. It is, however, difficult to prevent exfoliation of a bonding interface due to a thermal stress, the embrittlement and the exfoliation of the support member due to the active hydrogen.

Moreover, in the case of the solid- state targets each made of the lithium that are disclosed in patent documents 8- 12 given above, there are proposed the contrivance about the structure of the heat conductive plate serving as the support member of the lithium thin film and the method of coating the lithium particles with the refractory material in order to prevent the melting of the lithium (the melting point is approximately 180°C) having a low melting point. It is not, however, expected from these methods to tremendously improve the cooling efficiency, and it is considered difficult to prevent the lithium from being melted. For solutions of the problems described above, it is highly desired to solve the thermal problem of the target that arises due to the collision of the proton and to develop the target for reducing the radio activation of the member of the target etc due to the protons and the neutrons. None of the target capable of solving the problems given above is known at the status quo.

[Patent document 1]
Japanese Patent Application Laid-Open Publication No.H11-169470
[Patent document 2]
Japanese Patent Application Laid-Open Publication No. 2000-162399
[Patent document 3]
Japanese Patent Application Laid-Open Publication No. 2003-130997
[Patent document 4]
Japanese Patent Application Laid-Open Publication No. 2006-047115
[Patent document 5]
Japanese Patent Application Laid-Open Publication No. 2006-155906
[Patent document 6]
Japanese Patent Application Laid-Open Publication No. 2006-196353
[Patent document 7]
Japanese Patent Application Laid-Open Publication No. 2008-022920
[Patent document 8]
Japanese Patent Application Laid-Open Publication No. 2007-303983
[Patent document 9]
Japanese Patent Application Laid-Open Publication No. 2009-047432
[Patent document 10]
U.S. Patent No. 4,597,936
[Patent document 11]
International Publication Pamphlet No. WO 08/060663
[Patent document 12]

U.S. Patent Application No. 2010/0067640
[Non-patent document 1]
M. A. Lone, et. Al., Nucl. Instr. Meth. 143 (1977) 331.
[Non-patent document 2]
Title: "Neutronics of Coupled Liquid hydrogen Cold Neutron Source" authored by Yoshiyuki Kiyanagi, Hideki Kobayashi and Hirokatsu Iwasa, Bulletin of the Faculty of Engineering, Hokkaido University, 151:101-109 (1990 Jul. 30)
[Non-patent document 3]
Practice Manual of Calculation of Shielding Radiation Facilities, 2007, compiled by Nuclear Safety Technology Center, a Public Interest Incorporated Foundation
[Non-patent document 4]
JEBDL-4.0 (Japanese Evaluated Nuclear Data Library 4.0, published by Nuclear Data Center at Japan Atomic Energy Agency, Modified at 2010/09/29 17:22JST)
[Non-patent document 5]
K. Shibata, O. Iwamoto, T. Nakagawa, N. Iwamoto, A. Ichihara, S. Kunieda, S. Chiba, K. Furutaka, N. Otsuka, T. Ohsawa, T. Murata, H. Matsunobu, A. Zukeran, S. Kamada, and J. Katakura, "JENDL-4. 0 : A New Library for Nuclear Science and Engineering", J. Nucl. Sci. Technol. 48 (2011) 1-30.

## SUMMARY

It is an object of the present invention, in consideration of such circumstances, to provide a novel target for generating neutrons, a method of generating neutrons by use of the target and an apparatus for generating neutrons by use of the target which may generate neutrons by irradiation of low-energy protons, reduce radioactivation of the members including the target caused by protons and neutrons, and fundamentally solve a thermal problem of the target material and a problem of hydrogen embrittlement.

The present inventors, as a result of repeatedly making energetic researches for attaining the objects given above, found out that a target configured by compositing a beryllium material (or lithium material) and a nonmetal material, are highly effective as a target, and reached the completion of the present invention based on this knowledge.

Namely, one aspect of the present invention is a composite type target including:

1. A composite type target comprising:

   a target to generate neutrons by colliding protons with the target and to be configured by compositing a nonmetal material and one of a beryllium material and a lithium material.

2. The composite type target according to number 1, wherein the nonmetal material is a carbon-series material.
3. The composite type target according to number 2, wherein the carbon-series material includes at least one material of an isotropic graphite material and a crystal orientation carbon material.
4. The composite type target according to one of numbers 1 to 3, further comprising:

   a vacuum seal to be applied to a surface of the target and a cooling mechanism including a flow path for a coolant to be applied to a surface of the target.

5. A proton generating method of generating neutrons by colliding protons with a target, wherein:

   the protons are protons of equal to or larger than 2 MeV but smaller than 11 Mev,
   the target is the composite type target according to number 4, and
   the protons are collided with the composite type target under vacuum to generate neutrons by nuclear reactions and the target is cooled via the cooling mechanism of the composite type target.

6. A proton generating apparatus comprising:

   a hydrogen ion generating unit to generate protons;
   an accelerator to accelerate the protons generated by the hydrogen ion generating unit;
   a proton irradiating unit to irradiate the target with the protons accelerated by the accelerator; and
   a target to generate neutrons by colliding the protons with the target, wherein
   the accelerator is a linear accelerator, and
   the target is the composite type target according to number 4.

7. The proton generating apparatus according to number 6, wherein the linear accelerator accelerates protons in a range that is equal to or larger than 2 MeV but smaller than 11 MeV.

One aspect of the embodiment relates to a composite type target configured by compositing a beryllium material (or lithium material) and a nonmetal material. Also, one aspect of the embodiment relates to another composite type target configured by applying a vacuum seal to the above composite type target and collaterally fitting a cooling mechanism thereto. Functions of the composite type targets according to the present invention are "a reduction of the radioactivation of a member due to protons and neutrons" and "effective cooling of the target" in addition to a main function "the neutron generation based on nuclear reaction." The present invention relates to the composite type targets each configured by compositing two types of materials, and hence the functions of the target can be shared in terms of roles by the two types of materials. To be specific, one function is that the neutrons having a low energy can be generated by use of the protons having the low energy owing to properties with respect to protons possessed by the beryllium material (or lithium material). Another function is that it is feasible to remarkably reduce the radioactivation of the member such as the target due to protons and neutrons owing to properties with respect to protons and neutrons possessed by the nonmetal materials. Still another function is that the heat generated by the target can be promptly conducted to the surface of the target owing to excellent thermal diffusibility possessed by the nonmetal materials. Yet another function is that the composition of the beryllium material (or lithium material) and the carbon-series material enables the surface areas of these materials to be tremendously enlarged, i. e. , enables heat conduction areas to be tremendously enlarged, and it is therefore feasible to conduct the heat generated at the target to the surface of the target promptly. A further function is that the conducted heat is discharged outside the system through the cooling mechanism collaterally fitted to the target and formed with a coolant flow path, whereby the target can be efficiently cooled. Moreover, owing to this efficient cooling, secondary effects are acquired, such as being capable of using even low-melting lithium (a melting point: approximately 180°C) which has hitherto been difficult to be used as a solid-state target, preventing hydrogen embrittlement of the target material, preventing exfoliation at a bonding interface between the beryllium material (or lithium material) and the nonmetal material, and preventing blowout and fusion of beryllium (or lithium) even when employing beryllium (or lithium) thinner than beryllium (or lithium) hitherto used because of enabling the nonmetal material to function as a support material and a cooling material for the beryllium material (or lithium material).

With these effects, the composite type target according to the present invention solves the thermal problem of the target and can generate stably the neutrons exhibiting the low energy while reducing the radioactivation of the member such as the target.

Further, it is possible to use a linear accelerator defined as an accelerator, which is drastically downsized as compared with a conventional synchrotron or cyclotron serving as a source of generating the protons colliding with the composite type target of the present invention. Therefore, the medical neutrons for BNCT (Boron Neutron Capture Therapy) can be generated by providing the composite type target of the present invention in the small- sized linear accelerator that can be installed at a small- scale medical institution.

BRIEF DESCRIPTION OF THE DRAWINGS

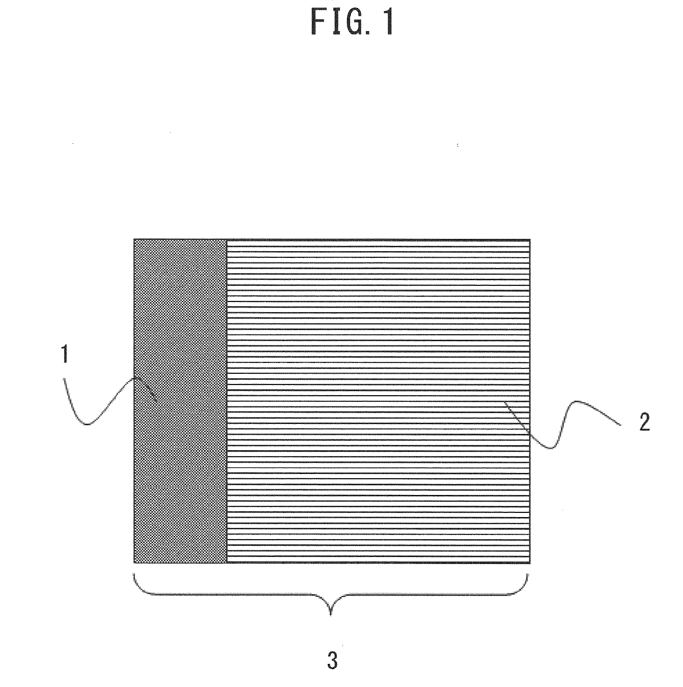

FIG. 1 is a sectional view illustrating a composite type target according to an embodiment in which the target is configuredbybondingaberylliummaterial (or lithium material) and a nonmetal material together.

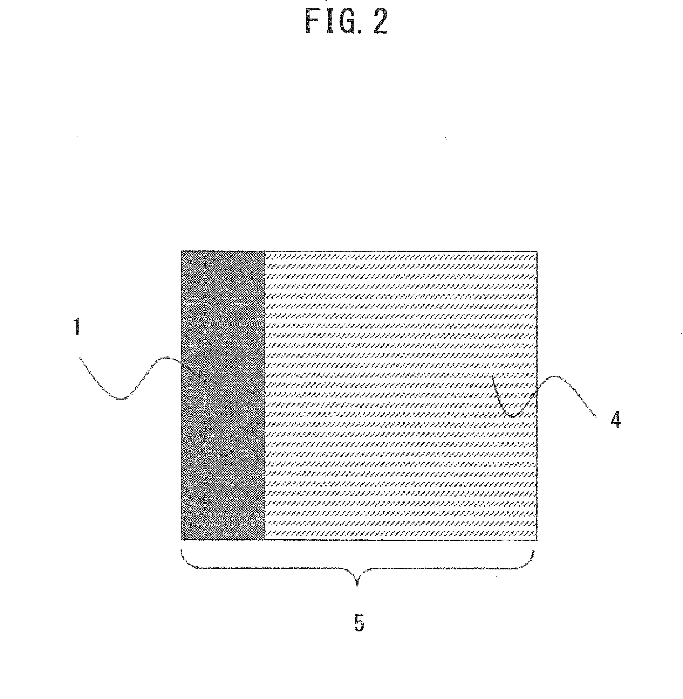

FIG. 2 is a sectional view illustrating a composite type target according to an embodiment in which the target is configured by bonding a beryllium material (or lithium material) and a nonmetal material together, and the nonmetal material is a carbon-series material which includes at least one material of an isotropic graphite material and a crystal orientation carbon material.

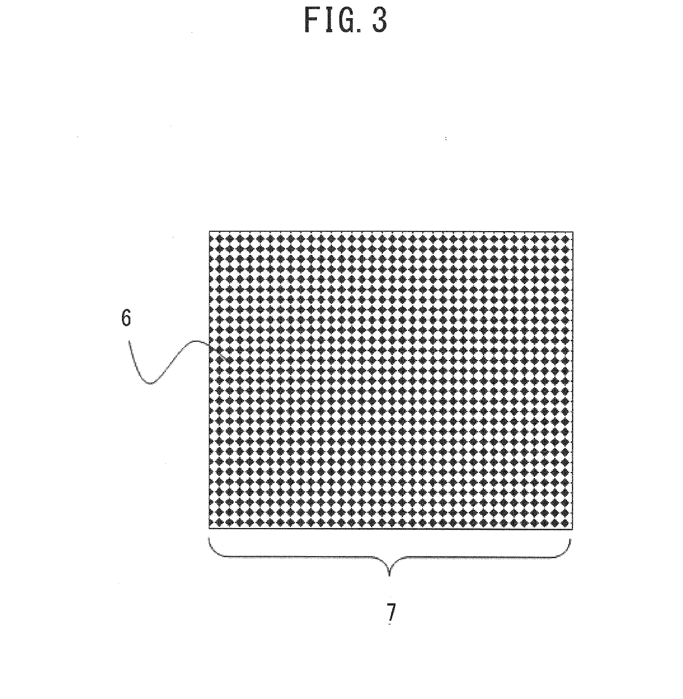

FIG. 3 is a sectional view illustrating a composite type target according to an embodiment in which a mixture of a beryllium material (or lithium material) and a nonmetal material is built up integrally.

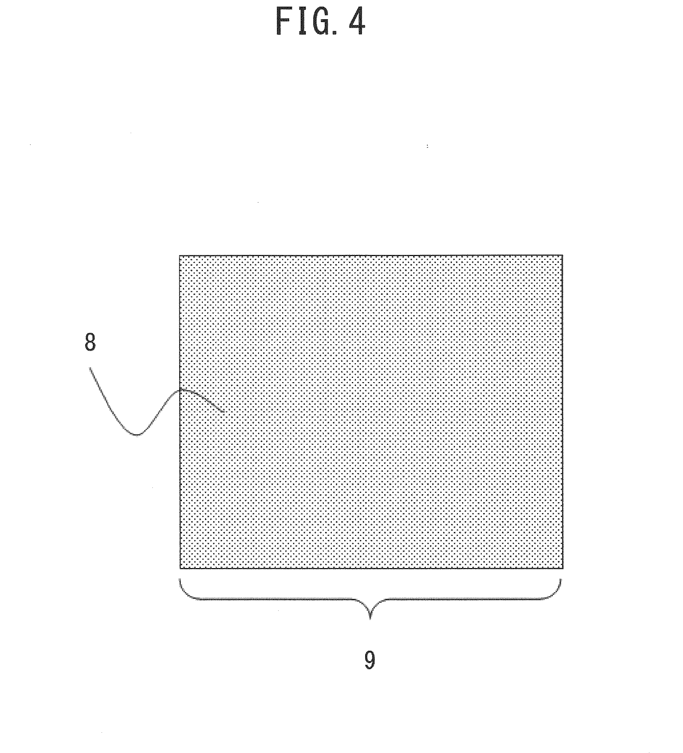

FIG. 4 is a sectional view illustrating a composite type target according to an embodiment in which a nonmetal material is molded with a beryllium material (or lithium material) dispersed.

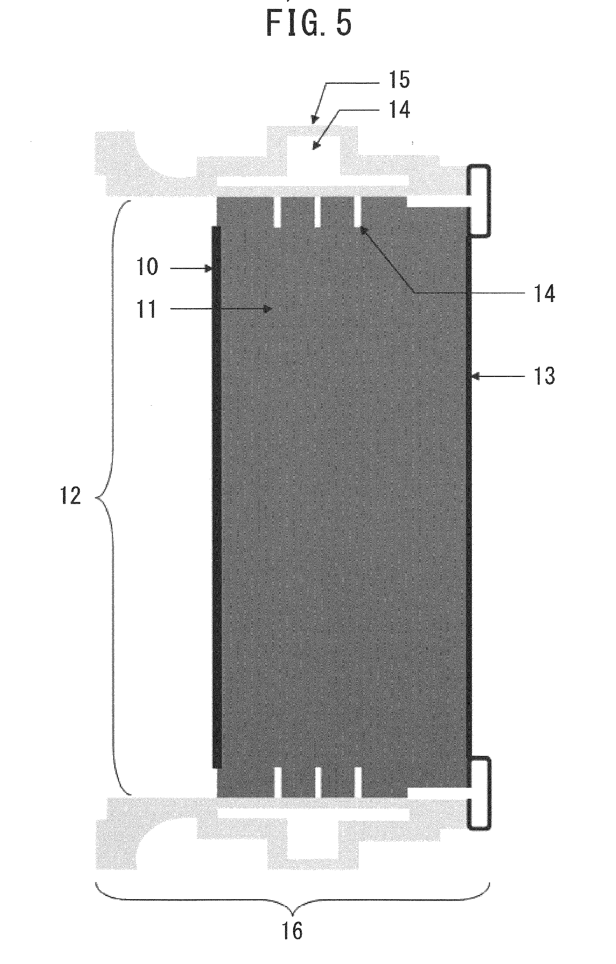

FIG. 5 is a sectional view illustrating a composite type target according to an embodiment in which the target is configured by bonding a beryllium material (or lithium material) and a nonmetal material together, a vacuum seal is applied to the surface of the target, and a cooling mechanism having a flow path for a coolant is collaterally fitted to the target.

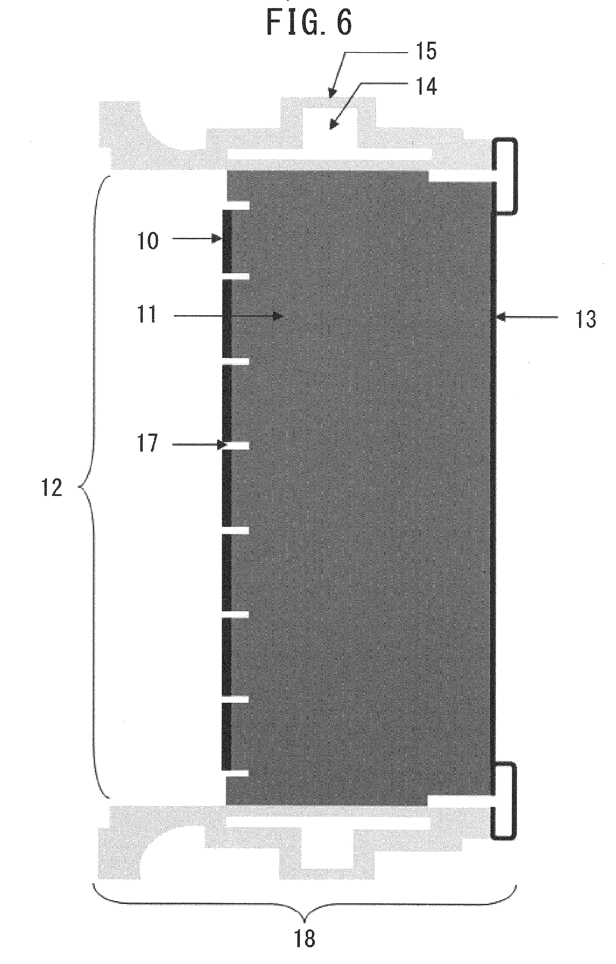

FIG. 6 is a sectional view illustrating a composite type target according to an embodiment in which the target is configuredbybondingaberylliummaterial (or lithium material) and a nonmetal material together, a vacuum seal is applied to the surface of the target, and a cooling mechanism having a flow path for a coolant is collaterally fitted to the target. Grooves are formed on one surface of the nonmetal material of the composite type target in order to partition and adhere the beryllium material (or lithium material) to the surface.

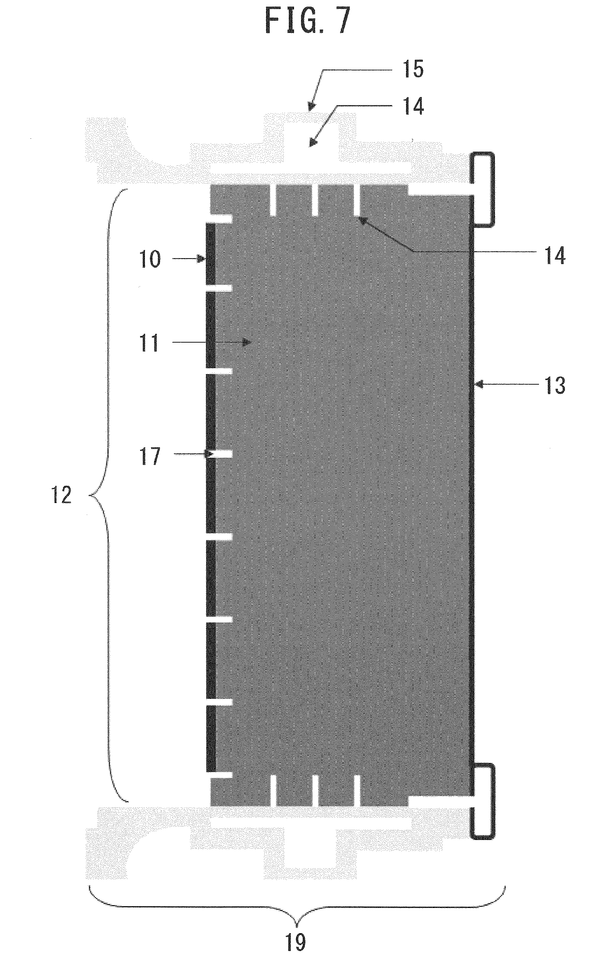

FIG. 7 is a sectional view illustrating a composite type target according to an embodiment in which the target is configured by bonding a berylliummaterial (or lithium material) and a nonmetal material together, a vacuum seal is applied to the surface of the target, a cooling mechanism having a flow path for a coolant is collaterally fitted to the target, and a independent flow path for a coolant is provided in the interior of the target. Grooves are formed on one surface of the nonmetal material of the composite type target in order to partition and adhere the beryllium material (or lithium material) to the surface.

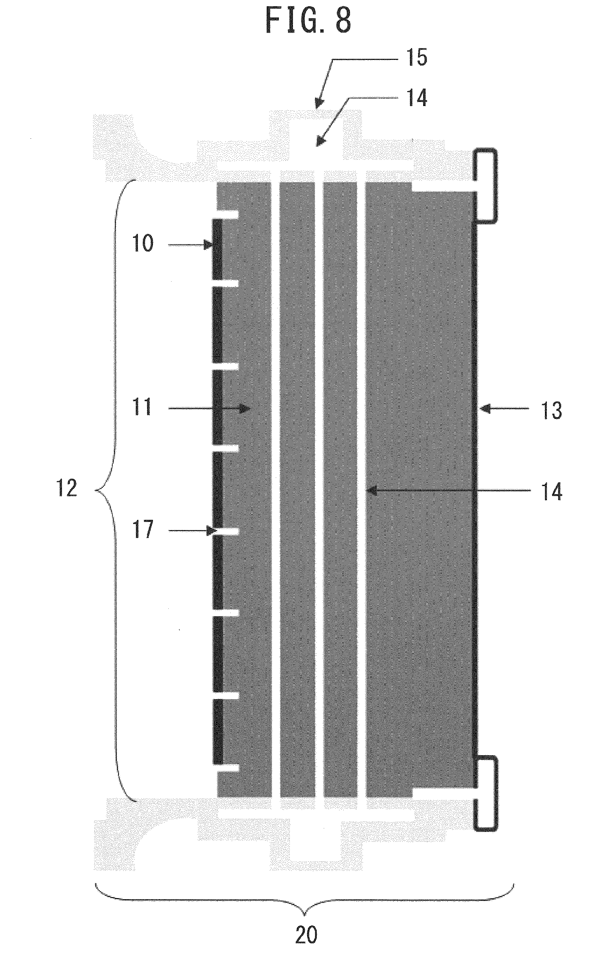

FIG. 8 is a sectional view illustrating a composite type target according to an embodiment in which the target is configured by bonding a beryllium material (or lithium material) and a nonmetal material together, a vacuum seal is applied to the surface of the target, a cooling mechanism having a flow path for a coolant is collaterally fitted to the target, a flow path for a coolant is provided in the interior of the target, and the internal flow path for the coolant is connected with the flow path for the coolant of the cooling mechanism. Grooves are formed on one surface of the nonmetal material of the composite type target in order to partition and adhere the beryllium material (or lithium material) to the surface.

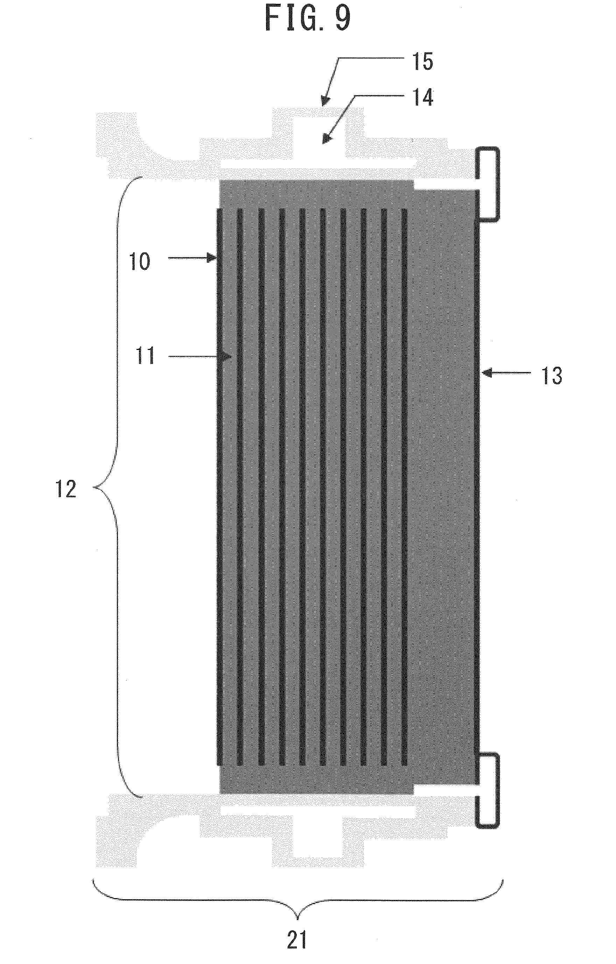

FIG. 9 is a sectional view illustrating a composite type target according to an embodiment in which the target configured by alternately stacking a layer of a beryllium material (or layer of a lithium material) and a layer of a nonmetal material on each other more than once, a vacuum seal is applied to the surface of the target, and a cooling mechanism having a flow path for a coolant is collaterally fitted to the target.

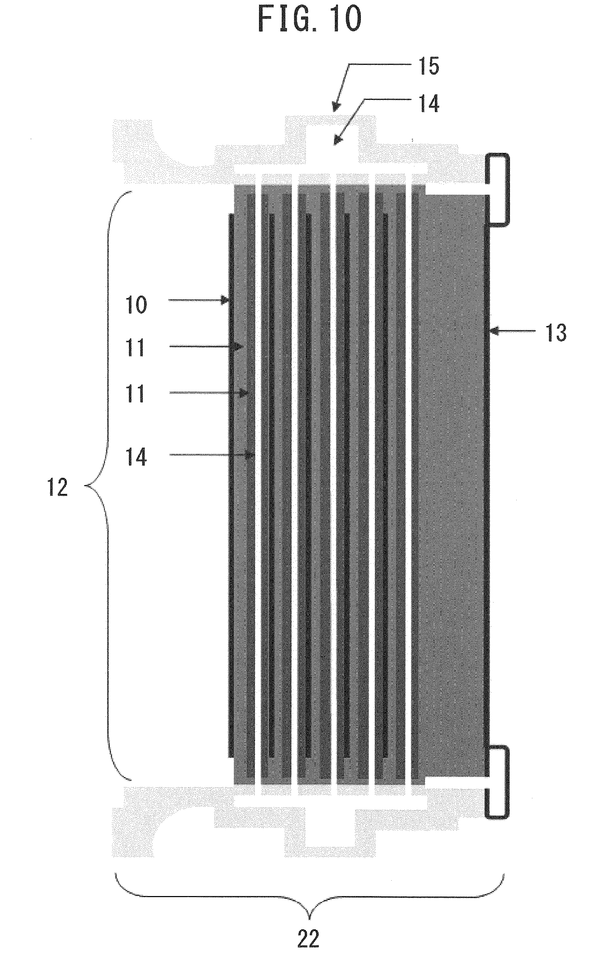

FIG. 10 is a sectional view illustrating a composite type target according to an embodiment in which the target is configuredbybondingaberylliummaterial (or lithium material) and a nonmetal material together to form a composite, and then alternately stacking a layer of the beryllium material (or layer of the lithium material) and a layer of the nonmetal material on each other more than once so that both materials are stacked with nonmetal material inserted in between, a vacuum seal is applied to the surface of the target, a cooling mechanism having a flow path for a coolant is collaterally fitted to the target, flow paths for coolants are provided in the interior of the inserted nonmetal materials between the composites, and the internal flow paths for the coolants are connected with the flow path for the coolant of the cooling mechanism.

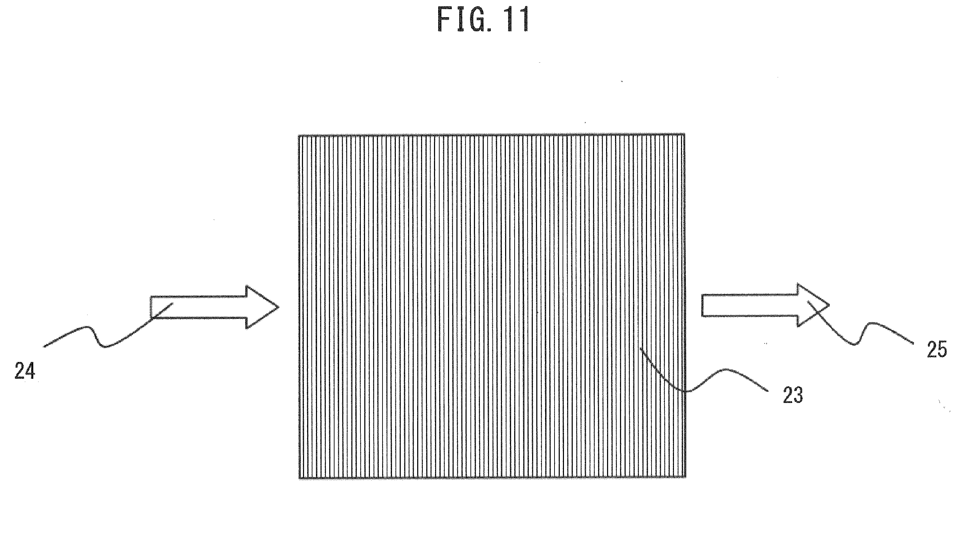

FIG. 11 is a schematic view illustrating a method for generating protons by use of a composite type target according to an embodiment of the present invention.

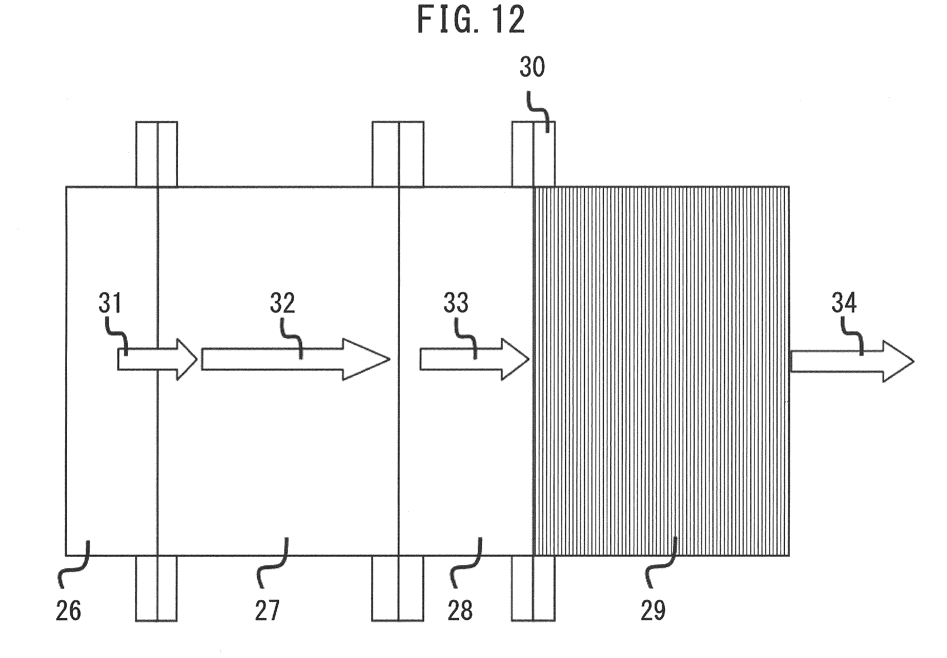

FIG. 12 is a schematic view illustrating an apparatus for generating protons by use of a composite type target according to an embodiment of the present invention.

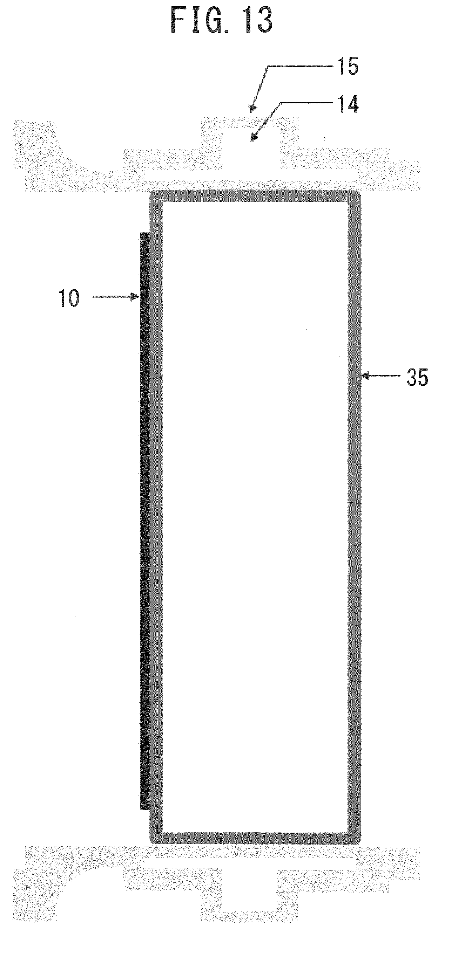

FIG. 13 is a sectional view illustrating a conventional target for comparison.

## DESCRIPTION OF EMBODIMENTS

As elucidated above, the main reason why the target according to the present invention is configured by compositing a beryllium material (or lithium material) and a nonmetal material, lies in sharing the functions of the target by the two types of materials described above. Specifically, the reason for using the beryllium material and the lithium material as the target material is mainly for generating neutrons having low energy through collisions with protons exhibiting low energy. In this connection, the beryllium material enables $^9$Be (p, n) reaction to occur by using protons of 4 MeV to 11 MeV, while the lithium material enables $^9$Li (p, n) reaction to occur by using protons of 2 MeV to 4 MeV.

Further, the reason for using the nonmetal material as another material of the target is chiefly for reducing the radioactivation due to protons and neutrons and for promptly conducting the heat generated at the target to the surface of the target by virtue of the high thermal diffusibility of the nonmetal material. Furthermore, it is because the nonmetal material, though having neutron generation efficiency that is smaller than that of the beryllium material (or lithium material), enables neutrons to be generated by collisions with protons.

In addition, the main reason why a carbon-series material which is selected among the nonmetal materials is used as a target material lies in the following features. That is, the carbon-series is particularly highly effective among the nonmetal material in reducing the radioactivation caused by protons and neutrons. Also, the carbon-series material has high durability against radioactive rays. In addition, the carbon-series material is small of absorption of thermal neutrons and epithermal neutrons. Furhter, the carbon-series material has a high neutron deceleration effect. Moreover, the carbon-series material shows similar or superior thermal diffusibility and thermal conductivity for diffusing and conducting the heat generated at the target comparing to metal materials. Additionally, the carbon-series material has a relatively high melting point. Thus, the carbon-series material is preferable for generating stably neutrons exhibiting low energy.

The beryllium material in the present invention represents a single element material of beryllium element (which is a simple substance metal of the beryllium element and hereinafter is referred to as beryllium) selected from within the elements of the second group of the periodic table, a beryllium compound, a beryllium alloy and a beryllium composite material. Moreover, the lithium material in the present invention represents a single element material of lithium element (which is a simple substance metal of the lithium element and hereinafter is referred to as lithium) selected from within the elements of the first group of the periodic table, a lithium compound, a lithium alloy and a lithium composite material. The reasons why the beryllium, the beryllium compound, the beryllium alloy and the beryllium composite material are generically termed the berylliummaterial and why the lithium, the lithium compound, the lithium alloy and the lithium composite material are generically termed the lithium material are that the principle of generating neutrons is based on nuclear reactions peculiar to specified elements. Namely, it is because the principle of generating neutrons by irradiating the target with accelerating protons is based on the physical nuclear reaction between protons and atoms of the specified element contained in the target, and therefore the neutrons are generated by the same nuclear reaction also when the target is composed of the compound of the specified element and the composite material as the case of the simple substance of the specified element. That is, according to the present invention, it is possible to use the beryllium compound, the beryllium alloy, the beryllium composite material, the lithium compound, the lithium alloy and the lithium composite material other than the beryllium and the lithium. When the compound and the composite material of the specified element as described above are used as the target material, it is desirable to use such a type of element that the elements excluding the specified elements (the beryllium element and the lithium element) contained in the compound and the composite material do not undergo the radioactivation by the protons and the neutrons and that a harmful substance is not generated due to the reaction to byproduct hydrogen atoms. These elements can be exemplified such as boron, carbon, silicon, nitrogen, phosphor, oxygen and sulfur, but are not limited to these elements.

As described above, the beryllium material according to the present invention represents beryllium, a beryllium compound, a beryllium alloy and a beryllium composite material. The beryllium compound can be exemplified such as beryllium halide including beryllium oxide ($BeO$), beryllium nitride ($Be_3N_2$), beryllium azide ($BeN_6$), beryllium phosphide ($BeP_2$), beryllium carbide ($Be_2C$), beryllium silicide ($Be_2Si$), beryllium fluoride ($BeF_2$), beryllium chloride ($BeCl_2$) and beryllium bromide ($BeBr_2$), beryllium hydroxide ($Be(OH)_2$), beryllium acetate ($Be(CH_3CO_2)_2$), beryllium carbonate ($BeCO_3$), beryllium sulfate ($BeSO_4$), beryllium nitrate ($Be(NO_3)_2$), beryllium phosphate ($Be_3(PO_4)_2$), beryllium silicate ($Be_2SiO_4$), beryllium aluminate ($Be(AlO_2)_2$), beryllium niobate ($Be(NbO_3)_2$) and beryllium tantalite ($Be(TaO_2)_2$), but is not limited to these materials. The beryllium alloy can be exemplified such as a magnesium beryllium alloy, an aluminum beryllium alloy and a lithium beryllium alloy, but is not limited to these alloys. Further, the beryllium composite material can be exemplified such as beryllium glass such as beryllium metaphosphate glass, beryllium glass ceramic containing beryllium glass as a main component, beryllium ceramic containing beryllium oxide as a main component, beryllium solution ceramic solved with the beryllium element and beryllium- doped endohedral fullerene, but is not limited to these materials. Among the beryllium materials given above, the beryllium and the beryllium oxide are most preferable because of having a high melting point (the melting point of the beryllium is approximately 1278°C, and the melting point of the beryllium oxide is 2570°C) though a threshold value (about 4 MeV) of the $^9$Be (p, n) reaction is comparatively high. The beryllium glass, the beryllium ceramic and the beryllium- doped endohedral fullerene are also preferable since the single substance of the beryllium is not eluted.

The lithium material according to the present invention represents lithium, a lithium compound, a lithium alloy and a lithium composite material. The lithium compound can be exemplified such as lithium halide including lithium oxide ($Li_2O$), lithium nitride ($Li_3N$), lithium carbide ($Li_4C$), lithium silicide ($Li_4Si$), lithium fluoride ($LiF$), lithium chloride ($LiCl$), lithium bromide ($LiBr$) and lithium iodide ($LiI$), lithium hydroxide ($LiOH$), lithium acetate ($LiCH_3CO_2$), lithium carbonate ($Li_2CO_3$), lithium sulfate ($Li_2SO_4$), lithium nitrate ($LiNO_3$), lithium phosphate ($Li_3PO_4$), lithium silicate ($Li_4SiO_4$), lithium aluminate ($LiAlO_2$), lithium niobate ($LiNbO_3$) and lithium tantalite ($LiTaO_2$), but is not limited to these materials. The lithium alloy can be exemplified such as a lithium magnesium alloy, a lithium aluminum alloy and a lithium beryllium alloy, but is not limited to these alloys. Furthermore, the lithium composite material can be exemplified such as lithium glass such as lithium silicate glass and lithium bisilicate glass, lithium glass ceramic containing the lithium glass as a main component, lithium ceramic containing lithium oxide as a main component, lithium solution ceramic solved with the lithium element and lithium- doped endohedral fullerene, but is not limited to these materials. Among the lithium materials given above, the lithium is most preferable because of having the low threshold value (about 2 MeV) of the $^7$Li (p, n) reaction though exhibiting a low melting point. The lithium glass, the lithium glass ceramic and the lithium- doped endohedral fullerene are also preferable since the single substance of the lithium is not eluted.

The nonmetal material according to the present invention is a material including at least one element which includes carbon and silicon, which are elements in Group 14 in the periodic table, nitrogen and phosphorus, which are elements in Group 15, oxygen and sulfur, which are elements in Group 16, and halogen which is in Group 17. The above elements are generically termed nonmetal elements. Namely, the nonmetal material according to the present invention is a single element material of a nonmetal element, a compound of a nonmetal element and a composite material of a nonmetal element. When the nonmetal element is carbon for example, the nonmetal material is a carbon material which is composed solely of carbon, a carbon compound which is composed of a carbon compound and a carbon-series composite material which is composed of more than one type of carbon material or carbon compound. Then, according to the present invention, the carbon material, the carbon compound and the carbon-series composite material are generically termed carbon-series material. Among these carbon-series materials, the carbon material can be exemplified such as isotropic graphite materials, crystal orientation carbon materials, polycrystalline diamond, diamond-like carbon, glassy carbon, porous carbon, polyacetylene carbon, carbynes, but is not limited to these materials. Moreover, among these carbon-series materials, the carbon compound can be exemplified such as carbon nitride and silicon carbide, but is not limited to these materials. Further, the carbon-series composite material can be exemplified such as carbon fiber reinforced plastic and carbon fiber reinforced ceramic, but is not limited to these materials. Among the carbon-series materials, preferable materials are isotropic graphite materials and crystal orientation carbon materials, which have well-balanced physical properties as described above, show superiority of thermal conductivity and thermal diffusibility but are hard to generate radioactive nuclides and also have, to be unexpected, a property of being hard to cause the hydrogen embrittlement. When the nonmetal element is silicon for example, the nonmetal material is a silicon material which is composed solely of silicon, a silicon compound which is composed of a silicon compound and a silicon-series composite material which is composed of more than one type of silicon material or silicon compound. The silicon material can be exemplified such as monocrystal silicon and polycrystal silicon, but is not limited to these materials. The silicon compound can be exemplified such as silicon dioxide ($SiO_2$), silicate, silica alumina and silicon nitride, but is not limited to these materials. The silicon-series composite materials can be exemplified such as sialon ceramics, silicon carbide ceramics and silicon nitride ceramics, but is not limited to these materials. In addition, since these silicon-series composite materials do not necessarily have high thermal conductivity, these silicon-series composite materials can be combined with a carbon-series material which has a high thermal conductivity.

The isotropic graphite material according to the present invention is a graphite material which has an isotropic structure and an isotropic characteristic. Generally, graphitic materials are classified into a CIP material (a compact into which a raw material of the graphite is molded in an isotropic way by Cold Isostatic Press), an extruded material and a mold material depending mainly on molding methods. The graphite material acquired via a graphitizing process after carbonizing the CPI material by baking is a graphite material having an isotropic structure and an isotropic characteristic and is therefore called the isotropic graphite material. Thus, the isotropic graphite material means herein a graphite material having an isotropic structure and an isotropic characteristic. The isotropic graphite material is preferable for the target material because of showing superiority such as having high thermal conductivity and being isotropic in terms of thermal conductivity in the same way as metal materials, having thermal diffusibility higher than that of metal materials, being hard to undergo radioactivation, being small of thermal neutrons and epithermal neutrons, having a high neutron deceleration effect, having durability against radioactive rays and exhibiting a high melting point (the melting point is approximately 3570°C). The usable graphite materials according to the present invention have a bulk density that normally falls within a range of 1.5 gcm$^{-3}$ to 3.5 gcm$^{-3}$. In the present invention, a graphite material, of which the bulk density is less than 1.5 gcm$^{-3}$, is not unusable, but, when the bulk density is less than 1.5 gcm$^{-3}$, it might happen that the collisions of carbon atoms with protons and neutrons become insufficient, and it is therefore preferable that the bulk density is more than or equal to 1.5 gcm$^{-3}$. Further, since a stable phase under the normal pressure is diamond when the bulk density exceeds 3.5 gcm$^{-3}$, the maximum value of the bulk density of the carbon material existing as substance is approximately 3.5 gcm$^{-3}$. An isotropic graphite material used as a conventional industrial material may be used as the isotropic graphite material in the present invention, and an isotropic graphite material improved to have a much higher density is more preferable.

The crystal orientation carbon material according to the present invention is a crystalline carbon material which is composed of carbon atoms or carbon molecules and in which crystals has the same orientation. Generally, crystallinity means that atoms and molecules constituting a material are arranged with a spatial repetition pattern, and orientation means that molecules and crystals are aligned in a direction. the meanings of crystal orientation follows this definitions. Namely, the crystal orientation carbon material according to the present invention is a crystalline carbon material which is composed of carbon atoms or carbon molecules and in which crystals has the same orientation. The crystal orientation carbon material can be exemplified such as single crystalline graphite, highly oriented pyloritic graphite (HOPG), carbon fibers, carbon nanofibers, vapor- grown carbon fibers (VGCF), carbon whiskers, carbon nanotubes, fullerenes, graphenes, single crystalline diamond and epitaxial diamond, but is not limited to these materials. In the single crystalline graphite, honeycomb layers (graphite layers) including a chain of six- membered rings of the carbon atoms (containing partially five- membered rings), which are strung in plane, are bonded by weak Van der Waals force to form layered structures. The layered structures are arranged with regularity like a crystal and the surfaces of graphite layers (hereinafter referred to as graphite surfaces) are oriented to the same direction in order. The HOPG is a graphite material which has a high crystal orientation similar to the single crystalline graphite although HOPG does not show a perfect crystal orientation as the single crystalline graphite shows. The carbon fibers, carbon nanofibers, VGCF and carbon whiskers are graphite materials in which microcrystals of graphite aggregate in a fibrous form and graphite layers are oriented to the direction of the fiber axis. The carbon nanotubes are carbon materials in which a cylindrical hollow is formed in the center of each molecule and one or more cylindrical graphite layers are formed so as to cover the hollow. The fullerenes are crystalline carbon materials with a polyhedron shape which are composed of six- membered rings and five- membered rings of carbon atoms. The graphenes are carbon materials which are composed of planate graphite layers, in which molecules form one or more layers. The single crystalline diamond is a diamond in which the crystal structures are connected without discontinuity. The epitaxial diamond is a film- like diamond crystal in which diamond crystals are grown on a crystal used as a substrate, in which the crystals are grown in alignment with the crystal face orientation of the underlying substrate. Among the crystal orientation carbon materials according to the present invention, the single crystalline graphite is such that a value of a coefficient of thermal conductivity on the surface (graphite surface) of the graphite layer is normally 1500 $Wm^{-1}K^{-1}$, and the diffusion coefficient of the heat (given by the coefficient of thermal conductivity per specific heat capacity) is approximately 3.4 $m^2h^{-1}$. On the other hand, the coefficient of thermal conductivity of copper well known as the metal material having the high thermal conductivity is 400 $Wm^{-1}K^{-1}$, and the diffusion coefficient of the heat is about 0.42 $m^2h^{-1}$. Accordingly, among the crystal orientation carbonmaterials according to the present invention, the single crystalline graphite, and HOPG, carbon fibers, carbon nanofibers, VGCF, carbonwhiskers, carbonnanotubes, fullerenes and graphenes having the crystallinity and orientation as equivalent to the single crystalline graphite are preferable as the thermal conductive material for conducting and diffusing the heat generated at the target to and over the target surface along the graphite surface more promptly than the metal material, and the isotropic graphite is preferable as the thermal conductive material similarly to the metal material exhibiting the high thermal conductivity. Further, the single crystalline diamond is such that a value of the coefficient of thermal conductivity is 2300 $Wm^{-1}K^{-1}$, and the diffusion coefficient of the heat is approximately 4.6 $m^2h^{-1}$. Hence, among the crystal orientation carbon materials according to the present invention, the single crystalline diamond and the epitaxial diamond having the high- crystallinity/ high- orientation equivalent thereto are preferable as the thermal conductive materials for promptly conducting and diffusing the heat generated at the target toward the target surface in the isotropic way (three- dimensionally) . The usable crystal orientation carbon materials according to the present invention have a bulk density that normally falls within a range of 1.5 $gcm^{-3}$ to 3.5 $gcm^{-3}$. In the present invention, the carbon material, of which the bulk density is less than 1.5 $gcm^{-3}$, is not unusable, however, if less than 1.5 $gcm^{-3}$, it might happen that the collisions among carbon atoms, protons and neutrons become insufficient, and it is therefore preferable that the bulk density is equal to or larger than 1. 5 $gcm^{-3}$. Further, if the bulk density exceeds 3.5 $gcm^{-3}$, a stable phase under the normal pressure is the diamond, so that the maximum value of the bulk density of the carbon material existing as the substance is approximately 3.5 $gcm^{-3}$. The crystal orientation carbon materials used as conventional industrial materials are usable as the crystal orientation carbon materials used in the present invention, and the carbon materials improved to have a much higher density are further preferable.

Moreover, the carbon- series materials in the present invention can be formed into the carbon- series composite material by compositing at least one of the isotropic graphite materials and the crystal orientation carbon materials among the carbon- series materials. Namely, preferable isotropic graphite materials or crystal orientation carbon materialsalone among the carbon- series materials may be used, or a composite carbon- series material in which an isotropic graphite material and a crystal orientation carbon material are composited may be used, or a composite carbon- series material in which an isotropic graphite material and a crystal orientation carbon material and another carbon- series material are composited may be used. The carbon- series material to be composited other than the isotropic graphite material and the crystal orientation carbon material may be one or plural carbon- series materials. These carbon- series materials can be exemplified by, as given above, the polycrystal diamond, the diamond- like carbon, the glassy carbon, the porous carbon, the polyacetylene, the carbynes, the carbon nitride and the silicon carbide, but are not limited to these materials. For example, the composite with the isotropic graphite materials and other carbon- series materials can be attained by bonding the compact of the isotropic graphite material to another compact of the other carbon- series material, mixing the isotropic graphite material with another carbon- series material, combining the isotropic graphite material with another carbon- series material, and so on. A component ratio of the isotropic graphite material is, though not particularly limited, normally equal to or larger than 50%. With this ratio being set, it is feasible to give a cooperative effect of the isotropic graphite material with another carbon- series material. For example, the thermal conductivity and the thermal diffusibility of the target can be further improved by compositing the isotropic graphite material with the diamond or carbon nanotube exhibiting the excellent thermal conductivity. In addition, for example, the composite of the crystal orientation carbon materials and the isotropic graphite materials may have isotropic thermal conductivity by alternately building up these materials.

Moreover, a reinforcing material can be properly added to the nonmetal material according to the present invention in order to improve the mechanical strength etc. when used. A preferable reinforcing material is a material that is hard to undergo the radioactivation. This type of materials can be exemplified such as epoxy resins, glass fibers and a variety of ceramic materials but are not limited to these materials.

It is known that an average energy of the neutrons generated at the target is about one- fifth of the energy of the incident protons (Non- patent document 1) . Accordingly, it is presumed that the average energy of the neutrons generated by colliding protons of 8 MeV with the beryllium material is approximately 1.6 MeV, and the average energy of the neutrons generated by colliding protons of 3 MeV with the lithiummaterial is approximately 0.6 MeV. The value of this average energy of the neutrons is within the energy range of fast neutrons, and hence the generated neutrons need to be decelerated down to the energy of thermal neutrons or epithermal neutrons by use of the decelerating material for the medical use such as the BNCT. The carbon- series materials such as light water ($H_2O$), heavy water ($D_2O$), beryllium

(Be), beryllium oxide (BeO) and graphite (C), as compared with such a point that a ferrous metal like iron, a nonferrous metal like copper and the heavy metal like tungsten exhibit almost no property of decelerating the neutrons, have a neutron deceleration ratio (which is a value obtained by dividing a value of moderating power of neutron by absorbing power of neutron, in which a larger value implies a better decelerating material) that is 1000 times as large as the decelerating ratio of the metal described above. Therefore, these carbon- series materials are generally employed as the neutron decelerating materials for a nuclear reactor etc. Among these materials, the carbon- series material such as graphite material has a neutron decelerating ratio that is larger than the neutron decelerating ratio of light water and has a neutron decelerating length (which is a migration length till the fast neutron is decelerated and becomes the thermal neutron, and is given as a square root of the Fermi age $\tau$cm$^2$) that is as comparatively short as about 20 cm (the value is approximately 4 times as large as the neutron decelerating length of the light water and is approximately twice as large as the neutron decelerating length of the heavy water) (Non- patent document 2), and is the suitable material as the neutron decelerating material for acquiring neutrons according to the present invention. Moreover, the carbon- series material such as the graphite is presumed to have the same neutron penetrability as the water has, and hence a neutron penetration ratio ($I/I_0$: a ratio of an intensity of the neutron after the penetration to an intensity of the incident neutron) in the carbon- series materials such as the graphite material is estimated to be about 60% on the basis of measurement data ($I/I_0 = 10^{-0.08T}$, where Tcm is the penetration length of the neutron, and the incident neutron has an energy of 1 MeV) (Non- patent document 3) of the neutron penetration ratio of the water on the assumption that a thickness of the carbon- series material like the graphite is, e.g., 3 cm. It is therefore presumed that the penetration of about 40% of the fast neutrons can be restrained. Further, for instance, if the thickness of the carbon- series material such as graphite materials is set equal to or larger than 20 cm, the penetration of the fast neutrons is restrained almost completely, and it is presumed that neutrons are acquired as thermal neutrons and epithermal neutrons.

The composite in the present invention embraces integrating the beryllium material (or lithium material) and the nonmetal material together. The specific aspect of the composite can be exemplified such as bonding the beryllium material (or lithium material) and the nonmetal material together, building up the beryllium material (or lithium material) and the nonmetal material together, mixing both materials together, combining both materials together, compatibilizing both materials together, compositing both materials based on surface working, dispersing the particles of one material to the other material, adhering one material to the other material, painting one material on the other material, but is not limited to these methods. In the composite type target according to the present invention, an interface is formed between the surfaces of the beryllium material (or lithium material) and the nonmetal material by compositing these two materials. The heat generated at the target is discharged in principle through the thermal conduction and the thermal diffusion at the interface between the materials, and hence the composite of the beryllium material (or lithium material) and the nonmetal material in the present invention is preferable. The interface is formed in a simple planar shape and a variety of complicated shapes, but a shape of a curved surface and a corrugated or rugged shape are preferable since the surface areas are larger than the surface area of a planar surface. When the nonmetal materials are carbon-series materials, in a target in which, for example, the berylliummaterial (or lithium material) and a carbon-series material having a good thermal conductivity such as a single crystalline graphite and isotropic graphite material are built up, the heat generated by the target can be promptly dissipated onto the surface of the target via the carbon-series material. For instance, in the target configured by molding a mixture of the powdered beryllium material (or lithium material) and the powdered carbon-series material, a specific surface area of the material can be made larger than the surface area of the bulk material depending on a particle size of the material, and it is therefore feasible to improve the thermal conductivity and the thermal diffusibility at the interface between the materials. For example, in the target containing the combination of the beryllium material (or lithium material) and the carbon-series material, the direct thermal conduction via the interface between the two materials can be done. Moreover, a shape of a curved surface and a corrugated or rugged shape can be formed on the target surface and the surface of the target material by the surface working over the target and the target material, thereby enabling the surface area of the target to be larger than the plane area thereof and enabling the thermal conductivity and the thermal diffusibility at the interface between the materials to be improved. The heat, which is thus conducted promptly to the target surface through the thermal conduction and the thermal diffusion, can be discharged outside the actual system by the indirect or direct cooling mechanism provided on the side surface, in the interior or on the bottom surface of the target, whereby the target can be cooled. Note that the specific surface area of the target is a total sum of the specific areas of the beryllium material (or lithium material) and the carbon-series material, which configure the target, and the plane area of the target connotes an area given when projecting the target surface on a parallel plane thereof. Further, the secondary effects of compositing the beryllium material (or lithium material) and the carbon-series material are given, such as improving adhesion between the beryllium material (or lithium material) and the carbon-series material, relaxing a thermal stress on the interface and preventing exfoliation on the interface.

As discussed above, the composite type target according to the present invention is capable of increasing the specific area of the target further than the plane area by compositing the beryllium material (or lithium material) and the nonmetal material together. The specific area of the target is, when positively enlarging this specific area, is roughly estimated to be, preferably, twice or more as large as the plane area of the target. If the specific area of the target is twice or more as large as the plane area of the target, the thermal conduction to the target surface is speeded up, and it is therefore preferable that the heat can be efficiently discharged without providing the large heat conductive plate on the target surface. For instance, the surfaces of the beryllium material and the lithiummaterial are formed with the corrugated or rugged shapes and grooves by use of the surface working method such as laser ablasion, whereby the specific area can be easily enlarged twice or more. The powdered beryllium material and the powdered lithium material are dispersed over the powdered nonmetal material and are molded in the target shape, whereby the specific area can be enlarged about 100 times. Furthermore, particles of the beryllium material and the lithium material are borne in minute holes of the porous nonmetal material by employing an impregnation method for adjusting a catalyst, whereby the specific area can be enlarged about 1000 times.

Concrete modes of the composite type target according to the present invention are exemplified as follows. For example, the beryllium material (or lithium material) and the nonmetal material are stacked together and thus molded into the target. The beryllium material (or lithium material) and the nonmetal material are stacked together and thus molded into the target. The surfaces of the beryllium material (or lithium material) and the nonmetal material are formed with the corrugated or rugged shapes and thus molded into the target. The mixture of the powdered beryllium material (or lithium material) and the powdered nonmetal material is molded into the target. The particles of the beryllium material (or lithium material) are dispersed into the porous nonmetal material, and the particle-dispersed material is molded into the target. The powdered nonmetal material is coated with the berylliummaterial (or lithium material), and the beryllium- or lithium-coated material is molded into the target. Both of the berylliummaterial (or lithium material) and the nonmetal material are combined and thus bonded and are molded into the target. The beryllium material (or lithium material) and the nonmetal material are adhered together via combination of both materials and are molded into the target. The mode of the composite type target is not, however, limited to those exemplified above. For instance, the surfaces of the beryllium material (or lithium material) and the nonmetal material are formed with the corrugated or rugged shapes and thus molded into the target, here the specific area of the target can be enlarged about several times. The specific area of the powdered material is by far larger than the specific area of the bulk material, so that the mixture of the powdered beryllium material (or lithium material) and the powdered nonmetal material is molded into the target, whereby the specific area of the target can be improved about 100 times as large as the plane area. For the same reason, the particles of the beryllium material (or lithium material) are dispersed into the porous nonmetal material, and the particle-dispersed material is molded into the target, whereby the specific area of the target can be enlarged about 1000 times as large as the plane area. Further, grooves and corrugated or rugged shapes may be formed on the surface of the target. This configuration may enlarge the specific area of the target and may acquire an effect in restraining excessive thermal concentration.

The method of compositing the target materials in the composite type target according to the present invention is properly determined corresponding to the composite modes, the types, the thicknesses, etc. of the materials to be used but is not limited to the specified working methods. For example, the composite based on bonding the beryllium material to the nonmetal material can be attained by hot pressing, an HIP (Hot- Isostatic- Pressing) process, evaporation, etc. In the case of stacking the comparatively thick beryllium material and the comparatively thick non metal material together, the hot pressing and the HIP process are preferable. In the case of stacking the comparatively thin beryllium material and the comparatively thin nonmetal material together, the evaporation is preferable. The beryllium material and the nonmetal material can be hot- pressed normally at a temperature ranging from 200°C up to the melting point of the beryllium material under the normal pressure and under a pressure ranging from $10^3$ kPa to $10^5$ kPa. The HIP process can be executed normally at a temperature ranging from 100°C up to the melting point of the beryllium material under the normal pressure and under a pressure ranging from $10^4$ kPa to $10^6$ kPa. The evaporation can be performed when a temperature of a substrate of the nonmetal material ranges from the room temperature up to the melting point of the beryllium material and under a pressure ranging from $10^{-3}$ Pa to $10^{-6}$ Pa. For instance, the beryllium and the nonmetal material are bonded together by the HIP process at 900°C or higher, and the beryllium carbide can be produced at the junction interface, thereby enabling the adhesive strength to be improved. Furthermore, for example, the composite based on stacking the lithiummaterial and the nonmetal material together can be attained by the hot pressing, the HIP process, the evaporation, etc. In the case of stacking the comparatively thick lithium material and the comparatively thick non metal material together, the hot pressing and the HIP process are preferable. In the case of stacking the comparatively thin lithium material and the comparatively thin nonmetal material together, the evaporation is preferable. The lithiummaterial and the nonmetal material can be hot- pressed normally at a temperature ranging from the room temperature (23°C) up to the melting point of the lithium material under the normal pressure and under a pressure ranging from $10^3$ kPa to $10^5$ kPa. The HIP process can be executed normally at a temperature ranging from the room temperature up to the melting point of the lithium material under the normal pressure and under a pressure ranging from $10^4$ kPa to $10^6$ kPa. The evaporation can be performed when a temperature of a substrate of the nonmetal material ranges from the room temperature up to the melting point of the lithium material and under the pressure ranging from $10^{-3}$ Pa to $10^{-6}$ Pa. The surface of the target and the surface of the target material can undergo the corrugated or rugged shaping process by the conventional methods such as the laser ablation, etching and die casting. The beryllium material and the lithium material can be coated over the nonmetal material by, e.g., a CVD (Chemical Vapor Deposition) method. The particles of the beryllium material and the lithium material can be dispersed into the nonmetal material by, e.g., the impregnation method for adjusting the catalyst. The coating based on the CVD method can be carried out by, e.g., a method of letting precursors of the gaseous beryllium material and the gaseous lithium material through the surface of the nonmetal material at a high temperature in an inactive atmosphere and depositing the berylliummaterial and the lithiummaterial by dint of thermal decomposition of the precursors. The particles of the beryllium material and the lithium material can be dispersed based on the impregnation into the nonmetal material by baking, after water solutions of the precursors of the beryllium material and the lithium material have been impregnated in the porous nonmetal material, the solution-impregnated nonmetal material in a reducing atmosphere and thus bearing the particles of the beryllium material and the lithium material in the minute holes of the nonmetal material. The materials can be powdered by the conventional methods such as mechanical milling, freezemilling, plasma atomizing and a spray drying method. When the mixed material is formed into the target, a binder and a sintering agent may be added as necessary. The binder and sintering agent are preferably a material which is not subj ect to radioactivation by protons and neutrons. The binder and sintering agent can be exemplified such as silicon dioxide, ceramics such as silica alumina, silicate and paste materials such as low- melting glass, but is not limited to these materials.

The manufacturing of compacts by compositing the composite type target according to the present invention is properly determined corresponding to the composite modes, the types, the thicknesses, etc. of the materials to be used but  is not limited to the specified working methods as described above. For example, a target in which a plurality of complexes of the beryllium material (or lithium material) and the nonmetal material are stacked together can be manufactured by stacking, a sheet prepared by evaporating the beryllium material (or lithiummaterial) onto the nonmetal material and a sheet prepared in a way that bonds the thin layer of beryllium material (or lithium material) by rolling to the nonmetal material so that the beryllium material (or lithium material) and the nonmetal material alternately abut on each other, and press-molding the stacked layers of these materials in the target shape by the hot pressing, the HIP process, etc. In addition, when the plurality of complexes are stacked, a layer of nonmetal material having a flow path for a coolant may be bonded between the stacked layers in order to cool each layer.

In the generation of neutrons due to the collisions between protons and the target, it is of much importance at all times how the heat generated at the target is efficiently discharged. Normally, the maximum value of the thermal load per unit surface area of the target is deemed to be a value obtained by dividing an output of the protons by the surface area of the target, and therefore a capacity of discharging the heat from the surface of the target must be designed to be equal or larger than the thermal load on the target. For example, the output of the protons needed for generating the neutrons for the medical use such as the BNCT is calculated to be at least 30 kW by way of a trial. Hence, supposing that the surface area of the target is 30 $cm^2$, it follows that the thermal load becomes 10 $MWm^{-2}$. The output of the protons is set, to be on the safe side, to a value to the greatest possible degree because a dosage of the generated neutrons becomes larger as the output gets higher. This being the case, however, one  type of target material has hitherto been used, and therefore, in the case of irradiating the target material having a surface area of 30 $cm^2$ with proton beams exhibiting an output of, e.g., 30 kW, there is proposed a cooling method involving an intermediary of a heat conductive plate having a larger surface area than the target material has (Patent document 6) because of its being difficult to perform direct cooling based on water cooling of the surface of the target material. According to this method, however, it is practically difficult to employ the solid target using the material such as the lithium exhibiting the low melting point. By contrast, the composite type target according to the present invention, which is configured by compositing the beryllium material or the lithium material with the carbon-series material, is capable of diffusing the heat via the carbon-series material and can therefore increase the output of the protons further than the conventional output value, whereby even the protons with the output of about 100 kW can be used. The composite type target according to the present invention is effective in solving the thermal problem of the target as described above.

The thicknesses of the beryllium material (or lithium material) in the composite type target according to the present invention can be made by far smaller than, though not particularly limited, a theoretical range of protons in the berylliummaterial (or lithium material) because the neutron generating reaction due to the collisions of the protons can be shared with the carbon-series material. The reason why so is that the nonmetal material functions as the support material and the cooling material for the beryllium material (or lithium material). Further, it is because the thermal loads burdened on the respective materials are reduced for the reason elucidated above. The theoretical range can be calculated from the incident  energy of the protons and stopping power of the substance. For example, when the target material is the beryllium, the theoretical range of the proton having the energy of 11 MeV in the beryllium is approximately 0.94 mm. Therefore, the conventional target composed of only the beryllium requires the thickness equal to or larger than 1 mm. The beryllium material in the target according to the present invention can be, however, made much thinner than 1 mm. When the beryllium material in the target according to the present invention is the beryllium, the thickness of the beryllium is preferably equal to or larger than 0.01 mm but smaller than 1 mm. Further preferably, the thickness of the beryllium is equal to or larger than 0.1 mm but equal to or smaller than 0.5 mm. If the thickness of the beryllium is smaller than 0.01 mm, the heat resistance remarkably declines, and hence it is preferable that the thickness of the beryllium is equal to or larger than 0.01 mm. Moreover, it is preferable for partly sharing the nuclear reaction due to the collisions of the protons with the beryllium that the thickness of the beryllium is smaller than 1 mm. Similarly, when the target material is the lithium, the theoretical range of the proton having the energy of 11 MeV in the lithium is approximately 2 mm. Hence, the conventional target composed of only the lithium requires the thickness equal to or larger than 2 mm. If the lithiummaterial in the target according to the present invention is the lithium, the thickness of the lithium can be, however, made much thinner than 2 mm. The thickness of the lithium in the target according to the present invention is preferably equal to or larger than 0.01 mm but equal to or smaller than 1 mm. Further preferably, the thickness of the lithium is equal to or larger than 0.05 mm but equal to or smaller than 0.5 mm. If the thickness of the lithium is smaller than 0.01 mm, the heat resistance declines, and hence it is preferable that the thickness of the lithium is equal to or larger than 0.01 mm. Moreover, it is preferable for partly sharing the nuclear reaction due to the collisions of the protons with the lithium that the thickness of the lithium is smaller than 1 mm. It is further preferable for keeping the heat resistance and for partly sharing the nuclear reaction due to the collisions of the protons with the lithium that the thickness of the lithium is equal to or larger than 0.05 mm but equal to or smaller than 0.5 mm.

The composite type target according to the present invention does not limit a ratio of the beryllium material (or lithium material) to the nonmetal material in the thicknesswise direction. The composite type target according to the present invention can adequately set this ratio corresponding to the target material to be used and the acceleration energy of irradiation protons, and normally sets the thickness of the nonmetal material ten times or more as large as the thickness of the beryllium material (or lithium material). The main reason why so is derived from a point that the neutron generation efficiency of the nonmetal material is smaller by one or more digits than the neutron generation efficiency of the beryllium material or the lithium material.

In the composite type target according to the present invention, the vacuum seal is applied to the target, and the cooling mechanism formed with the flow path for the coolant is collaterally fitted to the target. The chief reason for applying the vacuum seal to the target is that the target is irradiated with protons under the vacuum in the present invention and is therefore handled and manipulated under the vacuum. Further, the secondary effect yielded by applying the vacuum seal is for preventing the deterioration caused by the oxidation in the oxidative atmosphere when exposed to the atmospheric air. The vacuum seal may be a seal applied to only a portion exposed to the atmospheric air and may also be a seal applied to the target throughout. Sealing materials preferable for the vacuum seal are, though not particularly limited, light metal materials and the nonmetal materials because of having the property of being harder to undergo the radioactivation than heavy metals. The light metal materials can be exemplified such as magnesium, aluminum, tin, zinc, silicon, alloys of these light metal materials and a variety of ceramic materials, but are not limited to these materials. Further, the nonmetal materials can be exemplified such as glasses, epoxy resins and glass reinforced plastics, but are not limited to these materials.

Moreover, in the composite type target according to the present invention, the cooling mechanism formed with the flow path for the coolant is collaterally fitted to the target, and the main reason why so lies in that the target is cooled by actually discharging the heat generated at the target efficiently outside the system. The positions to which the cooling mechanism is applied for the composite type target are properly determined corresponding to the material constitutions and the required cooling ability etc. but are not limited to specific positions. When the target is manufactured by stacking the beryllium material (or lithium material) and the nonmetal material, the cooling mechanism can be provided on the side surface of the target, or on the bottom surface of the target. Alternatively, a flow path for a coolant can be formed in the interior of the nonmetal material. When the carbon- series materials such as the isotropic graphite material and the crystal orientation carbon material are used as nonmetal materials, it is preferable that the cooling mechanism is provided on the side surface of the target in order to use the high thermal conductivity on the orientation surface of the carbon- series material. For example, in case of a target in which the beryllium material (or lithium material) and the carbon- series material are alternately stacked, it is preferable that a flow path for a coolant is provided in the interior of the carbon- series material and a cooling mechanism having a common flow path for a coolant on the side surface of the target. The cooling mechanism is provided on the side surface of the composite type target, in which case the target can be cooled by the water via the heat conductive plate exhibiting the high thermal conductivity as the necessity may arise. In the case of providing the cooling mechanism on the bottom of the composite type target, it is preferable to use such a material as to cause almost no problem of the radioactivation caused by the neutrons. This type of material can be exemplified such as the carbon- series material according to the present invention. The preferable coolants in this case involve using, e. g., a liquid such as cooling water, and a gas such as gaseous helium having a high coefficient of thermal conductivity. Further, the composite type target according to the present invention can adopt a cartridge type structure in which the target and the cooling mechanism are built up integrally. This configuration enables the heat generated at the target to be efficiently discharged outside the system and enables the target to be easily detached and replaced with a new target through remote manipulation when the target gets deteriorated. In addition, with the advantages as described above, the composite type target according to the present invention may solve the thermal problems related to the target and can generate stably neutrons exhibiting low energy while reducing the radioactivation of the member of the target etc.

The neutrons generated by use of the composite type target according to the present invention are low-energy neutrons containing a large quantity of thermal neutrons or epithermal neutrons. The low-energy neutrons connote the neutrons in which the fast neutrons being harmful and exhibiting the high radioactivation are decreased. The fast neutrons have the energy that is higher by two digits than the thermal neutrons and the epithermal neutrons and are therefore biologically harmful and extremely high in terms of the radioactivation. The neutrons are classified into fast neutrons (also termed high speed neutrons), epithermal neutrons, thermal neutrons and cold neutrons. These neutrons are not, however, clearly distinguished in terms of the energy, and the energy classification differs depending on fields such as reactor physics, shielding, dosimetry, analysis and medical care. For instance, according to "Basic Glossary for Nuclear Emergency Preparedness", it says that "among the neutrons, the neutron having a large kinetic momentum is called the fast neutron (the high speed neutron), and the neutron called the fast neutron generally has a kinetic energy equal to or larger than 0.5 MeV, though this value differs depending on the fields such as the rector physics, the shielding and the dosimetry." Further, in the field of the medical care, the epithermal neutrons generally represent the neutrons within a range of 1 eV to 10 KeV, and the thermal neutrons generally connote the neutrons having the energy equal to or smaller than 0.5 eV. The low-energy neutrons defined in the present invention represent the neutrons in which the fast neutrons having the kinetic energy equal to or larger than 0.5 MeV are reduced. When the composite type target according to the present invention is irradiated with the protons of which the accelerating energy is equal to or larger than 2 MeV but equal to or smaller than 4 MeV, it is possible to generate the neutrons of which the average energy is about 0.3 MeV. Moreover, when the composite type target according to the present invention is irradiated with the protons of which the accelerating energy is equal to or larger than 6 MeV but equal to or smaller than 8 MeV, a quantity of the generation of the fast neutrons of 0.5 MeV or larger can be reduced by at least 30% against the conventional target composed of only the beryllium.

The neutrons can be generated by use of the composite type target according to the present invention and colliding protons of equal to or larger than 2 MeV but smaller than 11 MeV with this target under the vacuum. The collisions of protons with the composite type target under the vacuum are for preventing a decrease in intensity of the irradiation protons and preventing the air pollution. Accordingly, though under the high vacuum to be on the safety side, normally a degree of vacuum is within a range of $10^{-4}$ Pa through $10^{-8}$ Pa. In addition, the acceleration energy of the irradiation protons is set to equal to or larger than 2 MeV but smaller than 11 MeV in order to generate low energy protons in which fast protons are decreased. The acceleration energy of the proton needs to be properly set based on the types of the target materials building up the composite type target according to the present invention. In the case of using the beryllium material as the target material, the acceleration energy of the irradiation protons is preferably equal to or larger than 4 MeV but equal to or smaller than 11 MeV and further preferably equal to or larger than 6 MeV but equal to or smaller than 8 MeV. Since the threshold value of $^7$Be (p, n) reaction is about 4 MeV, if the acceleration energy of the protons is smaller than 4 MeV, the generation efficiency of the neutrons is remarkably decreased, and it is therefore preferable that the acceleration energy of the protons is equal to or larger than 4 MeV. Furthermore, if the acceleration energy of the protons exceeds 11 MeV, the radioactivation of the member such as the target gets conspicuous, and, in addition, a large quantity of fast neutrons occurs. It is therefore preferable that the acceleration energy of the protons is equal to or smaller than 11 MeV. The protons, which are further preferable for producing the low-energy neutrons with the reduction of the fast neutrons being harmful and exhibiting the high radioactivation, have the energy that is equal to or larger than 6 MeV but equal to or smaller than 8 MeV. Moreover, in the case of using the lithium material as the target material, the acceleration energy of the irradiation protons is preferably equal to or larger than 2 MeV but equal to or smaller than 4 MeV. Since the threshold value of $^7$Li (p, n) is about 2 MeV, if the acceleration energy of the protons is smaller than 2 MeV, the neutron generation efficiency remarkably decreases, and it is therefore preferable that the acceleration energy of the protons used in the present invention is equal to or larger than 2 MeV. Furthermore, if the acceleration energy of the protons exceeds 4 MeV, the radioactivation of the member such as the target gets conspicuous, and, in addition, the large quantity of fast neutrons occurs. It is therefore preferable that the acceleration energy of the protons is equal to or smaller than 4 MeV. The protons, which are further preferable for producing the low-energy neutrons with the reduction of the fast neutrons being harmful and exhibiting the high radioactivation, have the energy that is equal to or larger than 2 MeV but equal to or smaller than 4 MeV.

The neutrons can be generated by a neutron generating apparatus including the composite type target according to the present invention, a hydrogen ion generating unit for generating protons, an accelerator for accelerating the protons generated by the hydrogen ion generating unit, and a proton irradiating unit for irradiating the target with the protons accelerated by the accelerator. The neutron generating apparatus can be configured by providing a linear accelerator as the accelerator, using the composite type target according to the present invention as the target and disposing the composite type target in the proton irradiating unit. The hydrogen ion generating unit is provided with a hydrogen ion generator for generating the hydrogen ions. The hydrogen ion generator is not particularly limited but can involve using the conventional hydrogen ion generator. The generated hydrogen ions are transferred to the accelerator for the acceleration. The accelerator is, though being the linear accelerator, not particularly limited if being the linear accelerator but can involve employing the conventional linear accelerator. This type of linear accelerator can be exemplified such as a radio frequency quadrupole linear accelerator (RFQ linac), an electrostatic linear accelerator, a normal conduction linear accelerator, a super conducting linear accelerator and drift tube linac (DTL). The RFQ linac is a smaller-sized apparatus than the electrostatic linear accelerator nut can generate the protons of a large current. In addition, the RFQ linac produces an extremely small quantity of radioactive rays such as gamma rays and X-rays and is therefore preferable to the electrostatic linear accelerator. In addition, a DTL may be connected with the RFQ linac in a stage following the RFQ linac so that protons in the low or middle energy region may be further accelerated while converging the protons by use of electromagnets. Among the linear accelerators, the linear accelerator serving as a comparatively small-sized linear accelerator and capable of accelerating the protons in a range that is equal to or larger than 2 MeV but equal to or smaller than 11 MeV, is effective in generating the low-energy neutrons with the reduction of the fast neutrons being harmful and exhibiting the high radioactivation. The proton irradiating unit serves to irradiate the target with the protons accelerated by the accelerator and is normally provided with a proton beam adjusting means for converging, diffusing and scanning the protons and implementing the classification with respect to the target for generating the neutrons. The proton irradiating unit is not particularly limited but can involve using a conventional proton irradiating unit equipped with a quadrupole electromagnet or a bending electromagnet.

Further, it is possible to use a linear accelerator defined as an accelerator, which is drastically downsized as compared with a conventional synchrotron or cyclotron serving as a source of generating the protons colliding with the composite type target of the present invention. Therefore, the medical neutrons for BNCT (Boron Neutron Capture Therapy) can be generated by providing the composite type target of the present invention in the small- sized linear accelerator that can be installed at a small- scale medical institution.

An in-depth description of an embodiment (which will hereinafter be referred to as "the present embodiment") will be made by way of one aspect of the present invention with reference to the drawings.

The composite type target according to the present embodiment, which serves to generate the neutrons by colliding the protons with the target, is a composite type target in which a beryllium material (or lithium material) and a nonmetal material are composited and is configured by applying the vacuum seal to the surface of the target and collaterally fitting a cooling mechanism formed with a flow path for a coolant to the target. The respective materials of the target have a structure in which the materials are contiguous to each other via the interface. Available targets have the following forms. The targets can be exemplified such as a target configured by bonding the beryllium material (or lithium material) and the carbon-series material to each other, a target configured by molding a mixture of the powdered beryllium material (or lithium material) and the powdered carbon-series material, a target configured by molding the carbon-series material into which the particles of the beryllium material (or lithium material) are dispersed, and a target configured by combining and thus bonding both of the beryllium material (or lithium material) and the carbon-series material, but are not limited to these targets. The carbon-series material may be a single material and may also be a carbon-series composite material formed by compositing a plurality of carbon-series materials. Moreover, the composite type target can adopt such a cartridge type structure that the vacuum seal is applied to the target, and the cooling mechanism formed with the coolant flow path is collaterally fitted to the target. In addition, a groove may be applied to the surface of the composite type target in order to prevent the exfoliation of the beryllium material (or lithium material) and the carbon-series material of the composite type target as necessary. Also, a heat conductive plate may be provided at an intermediate portion between the cooling mechanism and the target according to the necessity.

A composite type target 3 according to the present embodiment illustrated in FIG. 1 is a composite type target taking such a form that a berylliummaterial (or lithium material) 1 and a nonmetal material 2 are bonded together. This type of composite type target may be manufactured as follows, for example. Namely, the beryllium material (or lithium material) and the nonmetal material 2 are hot-pressed under an inert gas atmosphere such as nitrogen gas at a temperature up to the melting points of the materials under a pressure of $10^4$ kPa to $10^6$ kPa. Preferable beryllium material (or lithium material) includes beryllium (or lithium), beryllium oxide (lithium oxide), beryllium glass (lithium glass) and beryllium glass ceramic (lithium glass ceramic). Preferable nonmetal material includes carbon-series materials such as isotropic graphite materials having high thermal conductivity, single crystalline graphite, HOPG, carbon fiber, single crystalline diamond and epitaxial diamond, and silicon-series materials such as single crystalline silicon, polycrystal silicon, silicon carbide and silicon niteride.

The composite type target 5 according to the present embodiment illustrated in FIG. 2 is a composite type target taking such a form that a beryllium material (or lithium material) 1 and a nonmetal material 4 are bonded together, and the nonmetal material 4 is a carbon-series material which includes at least one of an isotropic graphite material and a crystal orientation carbon material. This type of composite type target may be manufactured as follows, for example. Namely, the beryllium material (or lithium material) and the carbon-series material which includes at least one of an isotropic graphite material and a crystal orientation carbon material are hot-pressed under an inert gas atmosphere such as nitrogen gas at a temperature up to the melting points of the materials under a pressure of $10^4$ kPa to $10^6$ kPa. For example, a mixture of powdered isotropic graphite material (forexample, isotropic graphite powder having a bulk density of 2.2 gcm$^{-3}$ and the average particle size of 10 micron), industrial diamond powder (for example, diamond powder having the average particle size of 100 micron) and powdered carbon nanotube (for example, carbon nanotube having the average particle size of 10 micron) (for example, a mixture with the mass ratio of 0.8 : 0.1 : 0.1) is hot-pressed into a carbon compact under an inert gas atmosphere at a temperature up to the melting points of the materials under a pressure of $10^4$ kPa to $10^6$ kPa. This press enables the thermal conductivity of the carbon compact to be approximately twice as much as that of the isotropic graphite material. Next, a thick beryllium material (or lithium material), for example a beryllium film with the thickness from 0.1 mm to 0.5 mm or a lithium film with the thickness from 0.05 mm to 0.5 mm is hot-pressed onto one side of the carbon compact under an inert gas atmosphere at a temperature up to the melting points of the materials under a pressure of $10^4$ kPa to $10^6$ kPa to manufacture the composite type target. Since, in this type of composite type target, the smaller the particles of the nonmetal materials are the larger the specific surfaces of the materials are, the effects may be acquired such that the thermal conductivity area may be enlarged.

The composite type target 7 according to the present embodiment illustrated in FIG. 3 is a composite type target which is manufactured by integrally molding a mixture 6 of a beryllium material (or lithium material) 1 and a nonmetal material. This type of composite type target may be manufactured as follows, for example. Namely, a mixture of the beryllium material (or lithium material) and the nonmetal material are integrally molded under an inert gas atmosphere such as nitrogen gas at a temperature up to the melting points of the materials under a pressure of $10^4$ kPa to $10^6$ kPa. For example, a mixture of powdered beryllium (or lithium) (having the average particle size of 10 micron, for example) and powdered carbon nanotube (having the average particle size of 10 micron, for example) (forexample, a mixture with the mass ratio of 1: 100) is integrally molded into a composite type target under an inert gas atmosphere at a temperature up to the melting points of the materials under a pressure of $10^4$ kPa to $10^6$ kPa. This molding enables the specific surface of the beryllium (or lithium) component to be enlarged approximately 100 times. Other lithium material includes, for example, lithium- doped endohedral $C_{60}$ fullerene (having a lithium atom per one molecule of the fullerene), lithium disilicate glass ceramic ($Li_2O_2 \cdot SiO_2$) and lithium tantalate- alumina solid solution. Since, in this type of composite type target, the smaller the particles of the beryllium materials (or lithium materials) and the nonmetal materials are the larger the specific surfaces of the materials are, the effects may be acquired such that the thermal conductivity area may be further enlarged in comparison with the composite type target illustrated in FIG. 2.

The composite type target 9 according to the present embodiment illustrated in FIG. 4 is a composite type target which is manufactured by integrally molding a nonmetal material 8 in which the fine particles of a beryllium material (or lithium material) are dispersed. This type of composite type target may be manufactured as follows, for example. Namely, a nonmetal material in which the fine particles of the beryllium material (or lithium material) is integrally molded under an inert gas atmosphere such as nitrogen gas at a temperature up to the melting points of the materials under a pressure of $10^4$ kPa to $10^6$ kPa. For example, a porous carbon material as nonmetal material (for example, a carbon material having the average particle size of 200 micron, the specific surface of 600 $m^2$/g, the average pore size of 10 nanometer, a bulk density of 1.5 $gcm^{-3}$) is impregnated to bear nanoparticles of beryllium (or lithium) so as to adjust beryllium (or lithium) supported carbon material (having a supported rate of beryllium or lithium: 1 percent mass, for example), and then the beryllium (or lithium) supported carbon material is mixed with the same mass of powdered isotropic graphite material, for example, and then this mixture is integrally molded into the composite type target under an inert gas atmosphere at a temperature up to the melting points of the materials under a pressure of $10^4$ kPa to $10^6$ kPa. This molding of the supported material enables the specific surface of the beryllium (or lithium) component to be enlarged approximately 1000 times. Since, in this type of composite type target, the specific surface of the beryllium (or lithium) may be significantly enlarged, the effects may be acquired such that the thermal conductivity area may be also significantly enlarged in comparison with the composite type target illustrated in FIG. 3.

A composite type target 16 according to the present embodiment illustrated in FIG. 5 is a composite type target 12 taking such a form that a beryllium material (or lithium material) 10 and a nonmetal material 11 are bonded together, a vacuum seal 13 is applied to the surface of the nonmetal material 11 of the composite type target, a cooling mechanism 15 is formed with a flow path 14 for a coolant and collaterally fitted to the target, and the independent flow path 14 for the coolant is provided in the interior of the target. This type of composite type target may be manufactured as follows, for example. Namely, a beryllium material (or lithium material), which is for example a beryllium film with the thickness from 0.1 mm to 0.5 mm or a lithium film with the thickness from 0.05 mm to 0.5 mm, and a nonmetal material, which is for example a plate-like body, which is 165 mm in diameter and 30 mm in thickness, of a compact of a carbon-series material such as an isotropic graphite material and a crystal orientation carbon material is hot-pressed into the composite type target 12 under an inert gas atmosphere at a temperature up to the melting points of the materials under a pressure of $10^4$ kPa to $10^6$ kPa. The independent flow path 14 for the coolant is provided by for example preliminarily forming a groove on the side of the nonmetal material for running a cooling gas through therein. The vacuum seal 13 is applied by hot-pressing an aluminum film with the thickness of 0.1 mm for example onto the surface of the isotropic graphite material of the composite type target 12 under an inert gas atmosphere at a temperature up to the melting points of the materials under a pressure of $10^4$ kPa to $10^6$ kPa. Next, a cylindrical water cooling jacket for example, which is used as the cooling mechanism having the flow path 14 for the coolant, is soldered to the side of the composite type target 12 so as to manufacture the composite type target 16 which is unified with the cooling mechanism. Each composite type target illustrated in FIGS. 1 to 4 may be used as the composite type target 12. For example, the cylindrical water cooling jacket is capable of flowing 20-liter cooling water with the temperature of 5°C per minute at a flow velocity of 2 m per second. This corresponds to a cooling ability of approximately 100 kW. For example, cooled helium gas may pass through the independent flow path 14 for the coolant. Since, in this type of composite type target, the independent flow path 14 for the coolant is provided in the interior of the composite type target 12 in addition to the cooling mechanism 15, the cooling ability may be improved more than that of the single cooling mechanism 15.

A composite type target 18 according to the present embodiment illustrated in FIG. 6 is a composite type target 12 taking such a form that a beryllium material (or lithium material) 10 and a nonmetal material 11 are bonded together, a vacuum seal 13 is applied to the surface of the nonmetal material 11 of the composite type target, and a cooling mechanism 15 is formed with a flow path 14 for the coolant and collaterally fitted to the target. The composite type target 12 is formed with a groove 17 for partitioning and adhering the beryllium material (or lithium material) 10 to a single surface of the nonmetal material 11. This type of composite type target may  be manufactured as follows, for example. Namely, the composite type target 12 is manufactured in a similar way to manufacturing the composite type target illustrated in FIG. 5. As for the groove 17, cutting is preliminarily applied to one side of the nonmetal material which is for example a carbon-series material such as an isotropic graphite material and a crystal orientation carbon material to form a grid-like groove. Next, the composite type target is provided with the vacuum seal 13 and cooling mechanism as is the case with the composite type target illustrated in FIG. 5 to manufacture the composite type target 18 with which the cooling mechanism is unified. Since, in this type of composite type target, the groove is provided in order to partition and adhere the beryllium material (or lithium material) to a single surface of the nonmetal material, the effects may be acquired that exfoliation of the beryllium material (or lithium material) and the nonmetal material due to thermal stress may be prevented.

A composite type target 19 according to the present embodiment illustrated in FIG. 7 is a composite type target 12 taking such a form that a beryllium material (or lithium material) 10 and a nonmetal material 11 are bonded together, a vacuum seal 13 is applied to the surface of the nonmetal material 11, a cooling mechanism 15 is formed with a flow path 14 for the coolant and collaterally fitted to the target, and the independent flow path 14 for the coolant is provided in the interior of the target. The composite type target 12 is formed with a groove 17 for partitioning and adhering the beryllium material (or lithium material) to a single surface of the nonmetal material 11. This type of composite type target may be manufactured as follows, for example. Namely, the composite type target 12 is manufactured in a similar way to manufacturing the composite type target 12 illustrated in FIG. 5. The  independent flow path 14 for the coolant is provided as is the case with the composite type target 12 illustrated in FIG. 5. Next, the composite type target 12 is provided with the vacuum seal 13 and the cooling mechanism 15 having the flow path 14 for the coolant as is the case with the composite type target illustrated in FIG. 5 to manufacture the composite type target 19. Since this type of composite type target has the independent flow path 14 for the coolant in the interior of the composite type target 12 in addition to the cooling mechanism 15, the cooling ability may be further improved in comparison with the cooling mechanism of the composite type target illustrated in FIG. 6.

A composite type target 20 according to the present embodiment illustrated in FIG. 8 is a composite type target 12 taking such a form that a beryllium material (or lithium material) 10 and a nonmetal material 11 are stacked, a vacuum seal 13 is applied to the surface of the composite type target, a cooling mechanism 15 is formed with a flow path 14 for the coolant and collaterally fitted to the target, the independent flow path 14 for the coolant is provided in the interior of the target, and the internal flow path for the coolant is connected with the flow path for the coolant in the cooling mechanism 15. The composite type target 12 is formed with a groove 17 for partitioning and adhering the beryllium material (or lithium material) to a single surface of the nonmetal material 11. This type of composite type target may be manufactured as follows, for example. Namely, the composite type target 12 is manufactured in a similar way to manufacturing the composite type target illustrated in FIG. 5. For the flow path 14 for the coolant in the target which is connected with the flow path for the coolant in the cooling mechanism 15, cutting is applied in the nonmetal material which is for example a carbon-series material  such as an isotropic graphite material and a crystal orientation carbon material to so as to form a groove for the flow path 14 for the coolant. Next, the composite type target 12 is provided with the vacuum seal 13 and cooling mechanism 15 having the flow path 14 for the coolant as is the case with the composite type target illustrated in FIG. 5 to manufacture the composite type target 20. Since this type of composite type target has the independent flow path 14 for the coolant in the interior of the composite type target 12 in addition to the cooling mechanism 15, the cooling ability may be further improved in comparison with the cooling mechanism of the composite type target illustrated in FIG. 6. Cooling water which is commonly used for the cooling mechanism 15 may flow through the flow path 14 for the coolant connected with the cooling mechanism 15. Since, in this type of composite type target, the flow path 14 for the coolant connected with the cooling mechanism 15 is provided in the composite type target 12, direct cooling based on water cooling may be performed in the target. Thus, the cooling ability may be further improved in comparison with the cooling mechanism of the composite type target illustrated in FIG. 7.

A composite type target 21 according to the present embodiment illustrated in FIG. 9 is a composite type target 12 taking such a form that a plurality of beryllium materials (or lithium materials) 10 and nonmetal materials 11 are alternately stacked together, a vacuum seal 13 is applied to the surface of the composite type target, and a cooling mechanism 15 is formed with a flow path 14 for the coolant and collaterally fitted to the target. This type of composite type target may be manufactured as follows, for example. Namely, a beryllium material (or lithium material) (for example, a film with the thickness of 0.03 mm) is hot-pressed onto one side of a compact of a carbon-series material such as an isotropic graphite materials and a crystal orientation carbon materials (for example, a plate-like body which is 165 mm in diameter and 5 mm in thickness) under an inert gas atmosphere at a temperature up to the melting points of the materials under a pressure of $10^4$ kPa to $10^6$ kPa, and then a complex is manufactured. A plurality (for exapmle, ten sheets) of this complex is stacked in which both materials are stacked alternately is hot-pressed under an inert gas atmosphere at a temperature up to the melting points of the materials under a pressure of $10^4$ kPa to $10^6$ kPa, and then molded into the composite type target 12 (for example, a plate-like body which is 165 mm in diameter and 30 mm in thickness). Next, the vacuum seal and the cooling mechanism are applied to the composite type target 12 as is the case with the composite type target illustrated in FIG. 5 so as to manufacture the composite type target 21 which is unified with the cooling mechanism. Since, in this type of composite type target, the heat transmission area of the target material may be enlarged in proportion to the number of stacked target materials, the heat generated in the target may be transferred to the cooling mechanism more promptly than the composite type targets illustrated in FIGS. 5 to 8.

A composite type target 22 according to the present embodiment illustrated in FIG. 10 is a composite type target taking such a form that the structural features of the composite type targets illustrated in FIGS. 8 and 9 are united. Namely, the composite type target 12 is a composite type target in which a plurality of beryllium materials (or lithium materials) 10 and nonmetal materials 11 are alternately stacked together with carbon-series materials inserted in between, a vacuum seal 13 is applied to the surface of the composite type target, and a cooling mechanism 15 is formed with a flow path 14 for the coolant and collaterally fitted to the target, flow paths 14 for the coolant are provided in the nonmetal materials 11 between the above complex (corresponding to the structure of the flow path for the coolant in the composite type target illustrated in FIG. 8), and the internal flow path for the coolant is connected with the flow path 14 for the coolant in the cooling mechanism 15. This type of composite type target may be manufactured as follows, for example. Namely, cutting is applied to one side of the compact (for example, a plate-like body which is 165 mm in diameter and 5 mm in thickness) of the nonmetal material which is for example a carbon-series material such as isotopic graphite material and crystal orientation carbon material so as to form the flow path 14 for the coolant (for example, a cylindrical groove). Next, a crystal orientation carbonmaterial (for example, a carbon fiber sheet with the thickness of 1 mm) is adhered to the other side of the compact. Then, a beryllium material (or lithium material) (for example, a film with the thickness of 0.1 mm) is hot-pressed onto the surface of the crystal orientation carbon material under an inert gas atmosphere at a temperature up to the melting points of the materials under a pressure of $10^4$ kPa to $10^6$ kPa, and then a complex is manufactured. Five sheets of this complex is stacked in which both materials are stacked alternately are hot-pressed under an inert gas atmosphere at a temperature up to the melting points of the materials under a pressure of $10^4$ kPa to $10^6$ kPa, and then molded into the composite type target 12 (for example, a plate-like body which is 165 mm in diameter and 30 mm in thickness). Next, the vacuum seal and the cooling mechanism are applied to the composite type target 12 as is the case with the composite type target illustrated in FIG. 5 so as to manufacture the composite type target 22. Since, in this type of composite type target, the heat transmission area of the target material may be enlarged in proportion to the number of stacked target materials and direct cooling may be performed by use of the cooling water flowing through the flow path 14 for the coolant provided in the target, the cooling ability of the cooling mechanism may be improved in comparison with the composite type target illustrated in FIG. 9.

A neutron generating method which uses the composite type target according to the present embodiment illustrated in FIG. 11 is a neutron generating method in which protons 24 having predetermined acceleration energy (equal to or larger than 2 MeV but equal to or smaller than 11 MeV) are collided with a composite type target 23 under the vacuum, whereby low-energy neutrons 25 can be generated.

A neutron generating apparatus which uses the composite type target according to the present embodiment illustrated in FIG. 12 is a neutron generating apparatus which includes a hydrogen ion generating unit 26, a linear accelerator 27 and a proton irradiating unit 28 having a composite type target 29 are connected via flanges 30. Then, the proton irradiating unit 24 is provided with a hydrogen ion generator, in which generated hydrogen ions 31 are introduced into and accelerated by the linear accelerator 27. Protons 32 accelerated up to predetermined energy by the linear accelerator 27 are introduced into the proton irradiating unit 28 connected to a front end portion of the linear accelerator 27 and are collided with the composite type target 29 provided in the proton irradiating unit 28, thereby generating low-energy neutrons 34. The linear accelerator 27 is not particularly limited if being a linear accelerator capable of generating protons of which the energy is equal to or larger than 2 MeV but equal to or smaller than 11 MeV. Further, the proton irradiating unit 28 is normally provided with a quadrupole electromagnet or a bending electromagnet.

FIG. 13 illustrates a conventional target in which beryllium (or lithium) 10 is adhered to a metallic support member 35 and the metallic support member is provided with a cooling mechanism having a flow path 14 for the coolant. The thickness of the beryllium (or lithium) is normally about 1 mm. The support member is normally made of copper and the heat transmission area is 200 cm$^2$ at most.

Whether or not the melting and the radioactivation of the target material occur when a composite type target according to the present invention and a conventional target are irradiated with protons may be predicted by the heat calculations and theoretical calculations of radioactivation as described below. A premise condition is that accelerating protons having an output 30 kW to 8 MeV are irradiated to the composite type targets according to the present invention and the conventional targets under the vacuum of $10^{-6}$ Pa. The targets are cooled by introducing the 20-liter water of about 5°C per minute at a flow velocity of 2 m per second into the water cooling jacket soldered to the targets. This corresponds to a cooling ability of approximately 100 kW.

[Prediction for Presence of Melting in Target Material from Heat Calculations]

Heat calculations may predict the presence of melting of the beryllium materials (or lithium materials) occurred by the heat generated in the targets. The heat balance between the heat generated in a target and the heat dispersed by a thermal conductive material is given by formula 1.

$$\text{AMOUNT OF HEAT GENERATED PER UNIT TIME } Q(W) = \kappa \times S \times \alpha \quad (\text{FORMULA 1})$$

In this formula, the left term connotes the amount of heat generated in the target per unit time and the right term connotes the amount of heat dispersed via a thermal conductive material attached to the target. $\kappa$ connotes the thermal conductivity ($Wm^{-1}K^{-1}$) of the thermal conductive material, S connotes the heat transmission area ($m^2$) of the target, and $\alpha$ connotes the temperature gradient ($Km^{-1}$) of the thermal conductive material. From formula 1, $\alpha$ is given by formula 2.

$$\alpha = \frac{Q}{\kappa S} \quad (\text{FORMULA 2})$$

In formula 2, the value of Q is equal to the output of protons. Further, the value of $\kappa$ is assigned with a value of each thermal conductivity material. Here, when the output of protons is 30 kW and S is 1 $m^2$, the value of $\alpha$ is 75 $Km^{-1}$ when the thermal conductive material is metallic copper ($\kappa$ = 400 $Wm^{-1}K^{-1}$), 40 $Km^{-1}$ when the thermal conductive material is isotropic graphite material ($\kappa$ = 400 $Wm^{-1}K^{-1}$), 20 $Km^{-1}$ when the thermal conductive material is single crystalline graphite material or HOPG ($\kappa$ = 1500 $Wm^{-1}K^{-1}$), and 13 $Km^{-1}$ when the thermal conductive material is single crystalline diamond or epitaxial diamond ($\kappa$ = 2300 $Wm^{-1}K^{-1}$). Since, in the conventional targets, beryllium (or lithium) is adhered to a metal plate, the targets face limitations for enlarging the heat transmission areas of the targets and the heat transmission areas are about 200 $cm^2$ at most. Thus, in a conventional target, the value of $\alpha$ is 3750 $Km^{-1}$ (or 37.5 $Kcm^{-1}$). This value is the temperature difference at about 1 cm from the center of the thermal source (or the center of the target) in the thermal conductive material. Therefore, when a cooling mechanism is collaterally fit to a disk-shape target which 165 mm in diameter, the temperature difference ($\Delta T$) between the thermal source and the coolant of the cooling mechanism is approximately 309°C. Namely, since, in the case of a conventional target using lithium as the neutron generating material, the center temperature of the thermal source is way over the melting point of lithium (about 180°C), it is predicted that the melting of lithium may occur. Also, since, in the case of a conventional target using beryllium as the neutron generating material, the temperature exceeds the melting point of beryllium (1278°C) when the cooling is suspended for 25 minutes, it is predicted that the melting of beryllium may occur. On the other hand, when a carbon-series material having high thermal conductivity such as an isotropic graphite material, a single crystalline graphite material, HOPG, a single crystalline diamond and epitaxial diamond is used for the target material of the composite type target according to the present invention and a cooling mechanism is collaterally fit to a disk-shape target which is 165 mm in diameter, calculating in the same way as described above by assigning each thermal conductivity of each material to formula 2 may obtain $\Delta T$ = 165°C for the isotropic graphite material, $\Delta T$ = 82.5°C for HOPG and $\Delta T$ = 53. 6°C for the single crystalline diamond or the epitaxial diamond. Thus, it is not predicted that the melting of beryllium (or lithium) may occur. Moreover, since, in the present invention, the heat transmission area S ($m^{-2}$) may be from several times to 1000 times as large as that of the conventional targets, the temperature gradient $\alpha$ may be decreased inversely proportional to the heat transmission area and the temperature difference $\Delta T$ between the thermal source and the coolant of the cooling mechanism may also be decreased. In this way, the output of protons may be significantly improved, which is difficult in the neutron generating methods using the conventional targets.

Next, the time variation of the temperature in the target may be predicted by the heat conduction equation shown in formula 3. For convenience, one- dimensional partial differential equation is used here.

$$\frac{\partial T}{\partial t} = c^2 \frac{\partial^2 T}{\partial x^2} \quad \text{(FORMULA 3)}$$

In the formula, T, t, x and c connote temperature, time, position and thermal diffusivity, respectively. Solving formula 3 may obtain a general solution as shown in formula 4.

$$T = Ae^{-c^2\omega t}\sin\omega x + Be^{-c^2\omega t}\cos\omega x \quad \text{(FORMULA 4)}$$

Formula 4 means that the thermal relaxation process is represented by oscillation. When a boundary condition is assigned to formula 4, the relaxation time $\tau$ until a uniform temperature of the target is achieved is given by formula 5.

$$\tau = \frac{\lambda^2}{4\pi^2 c} \quad \text{(FORMULA 5)}$$

In the formula, $\lambda$ connotes a wave length (or a width of the non- uniform temperature) . When $\lambda$ is about the radius of the target, the value of $\tau$ is approximately 1.5 sec when the thermal conductive material is metallic copper (c = 0.42 $m^2h^{-1}$) . Therefore, when the thermal conductive material is metallic copper and a spot heat generation occurs, the target material may melt before a thermal equilibrium is achieved. On the other hand, when a carbon- series material having high thermal conductivity as described above is used as the target material of the composite type target according to the present invention, the thermal equilibrium may be achieved within milliseconds. Thus, it may be predicted that chances of the melting are low even when a spot heat generation occurs.

[Predictionfor Presence of Radioactivation in Target Material from Theoretical Calculations]

Theoretical calculations are performed in order to predict the presence of the radioactivation in a target material. The theoretical calculations are carried out in accordance with JENDL- 4.0 (Non- patent document 4) including data of the nuclear reaction sectional areas of nuclear reactions of neutrons and a calculation method by use of Q values of nuclear reactions (the difference of rest mass energies before and after a nuclear reaction is referred to as Q value) (Non- patent document 5) . Outlines of the calculation results will hereinafter be described. (1) The nuclear reaction caused by the collisions between protons of 8 MeV and beryllium is given such as $^9Be$ (p, $\gamma$) $^{10}B$, $^9Be$ (p, n) $^9B$, $^9Be$ (p, pn) $^8Be$, $^9Be$ (p, $\alpha$) $^6Li$, $^9Be$ (p, 2n) $^8B$, $^9Be$ (p, pn) $^8Be$ and $^9Be$ (p, 2p) $^8Li$, in which a radioactive half- life of each of these nuclides is short (shorter than or equal to 1 sec), and an effective dose equivalent rate constant $\Gamma_e$ (a measurement unit representing a degree of emission of the gamma rays caused by the radioactivation: $\mu S_v m^2 MBq^{-1}h^{-1}$) of each of these nuclides is "zero". (2) The nuclear reaction caused by the collisions between protons of equal to or smaller than 6 MeV and beryllium is given such as $^9Be$ (n, $\gamma$) $^{10}Be$, $^9Be$ (n, 2n) $^8Be$ and $^9Be$ (n, $\alpha$) $^6He$, in which the radioactive half- life of each of these nuclides is short (shorter than or equal to 1 sec), and the effective dose equivalent rate constant $\Gamma_e$ of each of these nuclides is "zero". Note that the reason why the acceleration energy of the neutrons is set equal to or smaller than 6 MeV is derived from a point that the maximum energy of the neutrons generated by the collisions between the protons of 8 MeV and the beryllium is 6.1MeV. (3) The nuclear reaction caused by the collisions between protons of 3 MeV and lithium is given such as $^6Li$ (p, $\gamma$) $^7Be$, $^6Li$ (p, $\alpha$) $^3He$, $^7Li$ (p, $\gamma$) $^8Be$, $^7Li$ (p, n) $^7Be$ and $^7Li$ (p, $\alpha$) $^4He$, in which the radioactive half- life of each of these generated radioactive nuclides excluding $^7Be$ is short, and the effective dose equivalent rate constant $\Gamma_e$ of each of these nuclides excluding $^7Be$ is "zero" or "0.00847." (4) The nuclear reaction caused by the collisions between neutrons of 3 MeV and lithium is given such as $^6Li$ (n, $\gamma$) $^7Li$, $^6Li$ (n, p) $^6He$, $^6Li$ (n, t) $^4He$, $^6Li$ (n, $\alpha$) $^3H$ and $^7Li$ (n, $\gamma$) $^8Li$, in which the radioactive half- life of each of these generated radioactive nuclides excluding tritium (t or $^3H$) is short, and the effective dose equivalent rate constant $\Gamma_e$ of each of these nuclides excluding the tritium was "zero" or "0.00847." (5) The elements producing the radioactive nuclides having comparatively long radioactive half- life and comparatively high effective dose equivalent rate constant $\Gamma_e$ due to the collisions between neutrons of 6 MeV and the respective elements in Group 0 elements and Group 1- 18 elements in the periodic table, are Sc, Ti, Mn, Fe, Co, Ni, Cu and Pt. Among these elements, the radioactive nuclides produced by the radioactivation of ferrous materials are $^{54}Fe$ (n, p) $^{54}Mn$ (the radioactive half- life is 312 days, $\Gamma_e$0.111), $^{54}Fe$ (n, $\alpha$) $^{51}Cr$ (the radioactive half- life is 27.7 days, $\Gamma_e$0.0046), $^{56}Fe$ (n, p) $^{56}Mn$ (the radioactive half- life is 2.58 hours, $\Gamma_e$0.203) and $^{58}Fe$ (n, $\gamma$) $^{59}Mn$ (the radioactive half- life is 44.6 days, $\Gamma_e$0.147) ; and the radioactive nuclides produced by the radioactivation of copper materials are $^{63}Cu$ (n, $\gamma$) $^{64}Cu$ (the radioactive half- life is 12.7 hours, $\Gamma_e$0. 0259), $^{63}Cu$ (n, $\gamma$) $^{60}Co$ (the radioactive half- life is 5.27 years, $\Gamma_e$0.305) and $^{65}Cu$ (n, p) $^{65}Ni$ (the radioactive half- life is 2.52 hours,

$\Gamma_e 0.0671$).

Table 1 illustrates the results of the theoretical calculations of the above radioactivation.

[TABLE 1]

| Premise Conditions for Theoretical Calculations | Production of Radioactive Nuclides and Prediction of Radioactivation |
|---|---|
| Proton: 8 MeV<br><br>Target: Composite of Beryllium and Graphite | ·No Production Radioactive Nuclides by Nuclear Reaction to Protons<br>·No Radioactivation of Graphite by Protons and Neutrons |
| Proton: 8 MeV<br>Target: Bonding of Beryllium and Metal (Copper, Iron, Stainless Steel, etc) | No Production Radioactive Nuclides by Nuclear Reaction to Protons, but Radioactivation of Sc, Ti, Mn, Fe, Co, Ni, Cu and Pt by Neutrons Occurs |
| Proton: 3 MeV<br>Target: Composite of Lithium and Graphite | No Radioactivation of Graphite by Protons and Neutrons |
| Proton: 3 MeV<br>Target: Bonding of Lithium and Metal (Copper, Iron, Stainless Steel, etc) | Production of Radioactive $^7$Be and Tritium due to Nuclear Reaction to Protons, and Radioactivation of Sc, Ti, Mn, Fe, Co, Ni, Cu and Pt by Neutrons Occurs |

Next, for each composite type target illustrated in FIG. 5 to 10 and the conventional target illustrated in FIG. 13, it may be derived from the results of the heat calculations and the theoretical calculations as described above that whether or not the melting and the radioactivation of the target occurs. The results thereof are illustrated in Table 2.

[TABLE 2]

| Target in FIG. | Result Derived from Calculation | |
|---|---|---|
| Thermal Equilibrium Temperature and Melting of Target Material | Radioactivation of Target | |
| Composite Type Target in FIG. 5 | App. 170°C No Melting | No Radioactivation |
| Composite Type Target in FIG. 6 | App. 170°C No Melting | No Radioactivation |
| Composite Type Target in FIG. 7 | App. 170°C No Melting | No Radioactivation |
| Composite Type Target in FIG. 8 | App. 170°C No Melting | No Radioactivation |
| Composite Type Target in FIG. 9 | App. 32.5°C No Melting | No Radioactivation |
| Composite Type Target in FIG. 10 | App. 40°C No Melting | No Radioactivation |
| Target with Beryllium Adhered to Metal Plate in FIG. 13 | App. 314°C No Melting | Radioactivation of Metal Plate as Support Member for Target |
| Target with Lithium Adhered to Metal Plate in FIG. 13 | App. 314°C Melting | Radioactivation of Metal Plate as Support Member for Target |

Next, it may be determined that whether or not the melting and the radioactivation of a target material occurs by attaching a composite type target according to the present invention to the neutron generating apparatus as illustrated in FIG. 11 and irradiating protons to the target. As a representative example, the composite type target 16 illustrated in FIG. 5 is fitted to the proton irradiating unit provided at the front end portion of the linear accelerator via the flange so that the target is set perpendicular to a proton moving direction, then the accelerating protons having an output of 30 kW to 2 MeV to 10 Mev are collided with the target under the vacuum of $10^{-6}$ Pa. The accelerating protons are generated by an RFQ linac and DTL. The target is cooled by introducing the 20- liter water of about 5°C per minute at a flow velocity of 2m per second into the water cooling jacket. This corresponds to a cooling ability of 100 kW. Further, helium gas of-200°C flows through the independent flow path for the coolant. The irradiation time duration is about one hour. After the experiment, the apparatus is stopped and left for one day, and then a survey meter is used to check whether or not the radioactivation occurs and visual observation is employed whether or not the melting occurs. Table 3 illustrates the results.

For comparison, the same experiment as described above is carried out with a neutron generating apparatus to which the conventional target illustrated in FIG. 13 is attached. Table 3 also illustrates the results of this experiment.

[TABEL 3]

| Target in FIG. | Thermal Equilibrium Temperature and Melting of Target Material | Radioactivation of Target |
|---|---|---|
| Composite Type Target with Beryllium Composited in FIG. 5 | No Melting | No Radioactivation |
| Composite Type Target with Lithium Composited in FIG. 5 | No Melting | No Radioactivation |
| Target with Beryllium Adhered to Copper Plate in FIG. 13 | No Melting | Radioactivation of Copper Plate as Support Member for Target |
| Target with Lithium Adhered to Copper Plate in FIG. 13 | Melting | Radioactivation of Copper Plate as Support Member for Target |

As described above, the present invention is a novel target for generating neutrons by colliding protons with the target. As described in the above embodiments, the target is capable of, because of the target unit being configured by compositing the beryllium material (or lithium material) and the nonmetal material, producing low-energy neutrons with the reduction of fast neutrons being harmful and exhibiting the high radioactivation, easily discharging the heat generated at the target, efficiently cooling because of the cooling mechanism being collaterally fitted to the target and taking the cartridge type structure in which the target and the cooling mechanism are built up integrally. Hence, the target has a characteristic that this target is provided at the front end portion of the proton irradiating unit and can be easily, when the target gets deteriorated, detached and replaced with the new target through the remote manipulation.

Moreover, as described above, the carbon- series material as the constructive material of the composite type target according to the present invention has the neutron decelerating effect, whereby the generation of the fast neutrons is reduced. With this configuration, in the embodiment discussed so far, the deceleration mechanism for decelerating the generated neutrons can be downsized.

Further, the irradiation protons are the comparatively low energy protons of which the accelerating energy is equal to or larger than 2 MeV but smaller than 11 MeV. Therefore, the effects are acquired, such as remarkably reducing the radioactivation of the member like the target etc. due to protons, restraining the generation of harmful fast neutrons and enabling accelerating protons to be generated by the small- sized linear accelerator.

Accordingly, the composite type target according to the present invention is effective as a neutron source of the neutron generating apparatus for the medical care, which can be installed at a small- scale medical institution and generates neutrons for the medical care such as the BNCT.

It was confirmed from the results given above that the composite type target according to the present invention may exhibit high thermal stability and reduce the radioactivation than by the conventional target.

INDUSTRIAL USAGE

The composite type target according to the present invention denotes the following characteristics. The composite type target is capable of, because of the target unit being configured by compositing the beryllium material or the lithium material and the carbon-series material, reducing the radioactivation of the member due to protons and neutrons, decreasing the generation of the fast neutrons because it is feasible to use protons having the energy that is comparatively lower than hitherto been, solving the thermal problem of the target owing to compositing the beryllium material (or lithium material) and the nonmetal material, discharging the heat generated at the target outside the actual system in the composite type target taking the cartridge type structure in which the target unit and the cooling mechanism are configured integrally, and detaching and replacing the target with the new target safely and easily through the remote manipulation when the target gets deteriorated. Moreover, the neutron generating method and the neutron generating apparatus using the composite type target according to the present invention are capable of generating low-energy neutrons in a way that employs the small-sized linear accelerator, and hence the composite type target according to the present invention is highly effective in generating neutrons for the medical care such as the BNCT.

DESCRIPTION OF REFERENCE NUMERALS

1    Beryllium material (or lithium material)

2    Nonmetal material

3    Composite type target

4    Carbon-series material including at least isotropic graphite material or crystal orientation carbon material

5    Composite type target

6    Compact of a mixture of beryllium material (or lithium material) and nonmetal material

7    Composite type target

8    Compact of nonmetal material in which beryllium material (or lithium material) is dispersed

9    Composite type material

10    Beryllium material (or lithium material)

11    Nonmetal material

12    Composite type target

13    Vacuum seal

14    Flow path for a coolant

15    Cooling mechanism

16    Composite type target

17    Groove

18    Composite type target

19    Composite type target

20    Composite type target

21    Composite type target

22    Composite type target

23    Composite type target

24    Flow of protons

25    Flow of neutrons

26    Hydrogen ion generating unit

27    Linear accelerator

28    Proton irradiating unit

29    Composite type target

30    Flange

31    Flow of hydrogen ions

32    Flow of accelerating protons

33    Flow of irradiating protons

34    Flow of generated neutrons

35    Support member made from metal material

**Claims**

1. A composite type target comprising:

   a target to generate neutrons by colliding protons with the target and to be configured by compositing a nonmetal material and one of a beryllium material and a lithium material.

2. The composite type target according to claim 1, wherein the nonmetal material is a carbon-series material.

3. The composite type target according to claim 2, wherein the carbon-series material includes at least one material of an isotropic graphite material and a crystal orientation carbon material.

4. The composite type target according to one of claims 1 to 3, further comprising:

   a vacuum seal to be applied to a surface of the target and a cooling mechanism including a flow path for a coolant to be applied to a surface of the target.

5. A proton generating method of generating neutrons by colliding protons with a target, wherein:

   the protons are protons of equal to or larger than 2 MeV but smaller than 11 Mev,
   the target is the composite type target according to claim 4, and
   the protons are collided with the composite type target under vacuum to generate neutrons by nuclear reactions and the target is cooled via the cooling mechanism of the composite type target.

6. A proton generating apparatus comprising:

a hydrogen ion generating unit to generate protons;
an accelerator to accelerate the protons generated by the hydrogen ion generating unit;
a proton irradiating unit to irradiate the target with the protons accelerated by the accelerator; and
a target to generate neutrons by colliding the protons with the target, wherein
the accelerator is a linear accelerator, and
the target is the composite type target according to claim 4.

7. The proton generating apparatus according to claim 6, wherein the linear accelerator accelerates protons in a range that is equal to or larger than 2 MeV but smaller than 11 MeV.

# FIG. 1

# FIG. 2

# FIG. 3

# FIG. 4

8

9

# FIG. 5

# FIG. 6

# FIG. 7

# FIG. 8

# FIG. 9

# FIG. 10

FIG. 11

FIG. 12

# FIG. 13

## INTERNATIONAL SEARCH REPORT

| | International application No. |
|---|---|
| | PCT/JP2011/077557 |

A. CLASSIFICATION OF SUBJECT MATTER
*H05H6/00*(2006.01)i, *A61N5/10*(2006.01)i, *G21K5/08*(2006.01)i, *H05H3/06*
(2006.01)i, *H05H9/00*(2006.01)i

According to International Patent Classification (IPC) or to both national classification and IPC

B. FIELDS SEARCHED

Minimum documentation searched (classification system followed by classification symbols)
H05H6/00, A61N5/10, G21K5/08, H05H3/06, H05H9/00

Documentation searched other than minimum documentation to the extent that such documents are included in the fields searched
Jitsuyo Shinan Koho       1922-1996   Jitsuyo Shinan Toroku Koho   1996-2012
Kokai Jitsuyo Shinan Koho   1971-2012   Toroku Jitsuyo Shinan Koho   1994-2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2009-204428 A  (Sumitomo Heavy Industries,<br>Ltd.),<br>10 September 2009 (10.09.2009),<br>paragraphs [0019], [0020], [0031], [0032]<br>& CN 101521981 A        & KR 10-2009-0092713 A | 1-2,4,6-7<br>5<br>3 |
| Y<br>A | JP 2009-47432 A  (Kyoto University),<br>05 March 2009 (05.03.2009),<br>paragraph [0013]<br>(Family: none) | 5<br>1-4,6-7 |
| A | JP 64-35898 A  (Science Research Laboratory,<br>Inc.),<br>06 February 1989 (06.02.1989),<br>page 474, upper left column<br>& US 4812775 A        & EP 300105 A2 | 1-7 |

☐ Further documents are listed in the continuation of Box C.       ☐ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| 31 January, 2012 (31.01.12) | 07 February, 2012 (07.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H11169470 B
- JP 2000162399 A
- JP 2003130997 A
- JP 2006047115 A
- JP 2006155906 A
- JP 2006196353 A
- JP 2008022920 A
- JP 2007303983 A
- JP 2009047432 A
- US 4597936 A
- WO 08060663 A
- US 20100067640 A

### Non-patent literature cited in the description

- **M. A. LONE.** *Nucl. Instr. Meth.,* 1977, vol. 143, 331
- **YOSHIYUKI KIYANAGI ; HIDEKI KOBAYASHI ; HIROKATSU IWASA.** Neutronics of Coupled Liquid hydrogen Cold Neutron Source. *Bulletin of the Faculty of Engineering,* 30 July 1990, vol. 151, 101-109
- Practice Manual of Calculation of Shielding Radiation Facilities. Nuclear Safety Technology Center, 2007
- Japanese Evaluated Nuclear Data Library 4.0. Nuclear Data Center at Japan Atomic Energy Agency, 29 September 2010, vol. 17
- **K. SHIBATA ; O. IWAMOTO ; T. NAKAGAWA ; N. IWAMOTO ; A. ICHIHARA ; S. KUNIEDA ; S. CHIBA ; K. FURUTAKA ; N. OTSUKA ; T. OHSAWA.** JENDL-4. 0 : A New Library for Nuclear Science and Engineering. *J. Nucl. Sci. Technol.,* 2011, vol. 48, 1-30